# EUROPEAN PATENT APPLICATION

(11) **EP 4 011 918 A1**
(43) Date of publication of application: **15.06.2022**
(21) Application number: 20849041.7
(22) Date of filing: 07.08.2020
(51) Int. Cl.: C07K 16/46, A61K 39/395, A61P 37/02, A61P 37/06, C07K 16/28, C12N 15/13

(54) **DUAL-SPECIFIC PROTEIN**

(30) Priority: 08.08.2019 JP 2019146227
(71) Applicant: ONO Pharmaceutical Co., Ltd., Osaka-shi, Osaka 541-8526 (JP)
(72) Inventor: SHIBAYAMA, Shiro, Tsukuba-shi, Ibaraki 300-4247 (JP); TEZUKA, Tomoya, Tsukuba-shi, Ibaraki 300-4247 (JP)
(74) Representative: Grünecker Patent- und Rechtsanwälte PartG mbB
(86) International application number: PCT/JP2020/030304
(87) International publication number: WO 2021/025140

(57) **Abstract**

The present invention addresses the problem of providing a new drug that, with respect to autoimmune diseases or blood cancer, is for preventing, suppressing the advance of symptoms, suppressing recurrence, or treating such diseases. The inventors of the present invention conducted extensive studies, and came to focus on a PD-1/CD4 dual-specific protein as a substance that can solve the foregoing problem, leading to completion of the present invention. The PD-1/CD4 dual-specific protein according to the present invention is configured to reduce the induction of generation or release of cytokine that occurs when the protein is administered. As a result, it can be expected that infusion reaction or cytokine release syndrome that could occur upon administration of the protein is suppressed.

SEQUENCE LISTING

<110>  ONO PHARMACEUTICAL CO., LTD.

<120>  Bispecific Antibody

<130>  P20-140WO

<150>  JP 2019-146227
<151>  2019-08-08

<160>  2

<170>  PatentIn version 3.5

<210>  1
<211>  5
<212>  PRT
<213>  Artificial Sequence

<400>  1

Gly Gly Gly Gly Ser
1               5


<210>  2
<211>  15
<212>  PRT
<213>  Artificial Sequence

<400>  2

Gly Gly Gly Gly Ser Gly Gly Gly Gly Ser Gly Gly Gly Gly Ser
1               5                   10                  15

## Description

### [Technical Field]

The present disclosure relates to a bispecific protein capable of specifically binding to PD-1 and CD4, respectively (hereinafter, may be abbreviated as a "PD-1/CD4 bispecific protein"), and a pharmaceutical therapeutic use thereof.

### [Background Art]

PD-1 is an immunosuppressive receptor belonging to an immunoglobulin family, and a molecule having a function of suppressing the immune activation signals of T-cells activated by stimulation through an antigen receptor. From analysis of PD-1 knock-out mice or the like, it is known that PD-1 signals play important roles in suppression of autoimmune diseases such as autoimmune dilated cardiomyopathy, lupus-like syndrome, autoimmune encephalomyelitis, systemic lupus erythematosus, graft-versus-host disease, type I diabetes mellitus, rheumatoid arthritis and the like. Accordingly, it is pointed out that a substance enhancing the PD-1 signal could be a prophylactic or therapeutic agent for autoimmune diseases.

All subtypes of T cells express the CD3 antigen on their surfaces. On the other hand, the expression patterns of other antigens differ among subtypes. For example, in addition to CD3, killer T cells express CD8, helper T cells express CD4, and regulatory T cells express CD4, CD25, FoxP3, and CD127, respectively.

It is known so far that there are bispecific antibodies recognizing PD-1 as a substance enhancing the PD-1 signal (Patent Literatures 1 to 3). These bispecific antibody are in the form which an antigen-recognition site of an antibody recognizing CD3, which is a member of a T-cell receptor complex, and an antigen-recognition site of an antibody recognizing PD-1 are linked to each other in genetic engineering method, and has an activity to enhance the inhibitory signal of PD-1 against the T-cell receptor complex by increasing the frequency of bringing PD-1 to the vicinity of the T-cell receptor complex. Furthermore, the Patent Literatures also state that the PD-1 bispecific antibody can be used for preventing or treating autoimmune diseases.

However, there is no report about the PD-1/CD4 bispecific protein of the present invention.

### [Citation List]

### [Patent Literature]

Patent Literature 1: International Publication No. WO2003/011911
Patent Literature 2: International Publication No. WO2004/072286
Patent Literature 3: International Publication No. WO2013/022091

### [Summary of Invention]

### [Technical Problem]

The object of the present invention is to provide a new pharmaceutical agent for preventing, suppressing the progression of symptoms of or the recurrence of or treating autoimmune diseases, hematological cancer and the like.

### [Solution to Problem]

The inventors of the present invention diligently studied and focused on the PD-1/CD4 bispecific protein as a substance capable of solving the above-mentioned problem, and then completed the present invention.

That is, the present invention relates to the followings:
[1] A bispecific protein having a first arm specifically binding to PD-1 and a second arm specifically binding to CD4 (in the present specification, being synonymous with a "bispecific protein capable of specifically binding to PD-1 and CD4, respectively", which may also be abbreviated as the "PD-1/CD4 bispecific protein.").
[2] A bispecific antibody (hereinafter, may be abbreviated as a "PD-1/CD4 bispecific antibody") or an antibody fragment thereof, having a first arm specifically binding to PD-1 (hereinafter, may be abbreviated as "the first arm") and a second arm specifically binding to CD4 (hereinafter, may be abbreviated as "the second arm") (hereinafter, may be collectively abbreviated as a "PD-1/CD4 bispecific antibody and the like").
[3] The PD-1/CD4 bispecific antibody or antibody fragment thereof according to the preceding item [2], wherein the first arm contains a heavy chain variable region (hereinafter, be abbreviated as a "VH") and a light chain variable region (hereinafter, be abbreviated as a "VL") of an antibody specifically binding to PD-1 (hereinafter, be referred to as an "anti-PD-1 antibody").
[4] The PD-1/CD4 bispecific antibody or antibody fragment thereof according to the preceding item [2] or [3], wherein the second arm contains a VH and VL of an antibody specifically binding to CD4 (hereinafter, be referred to as an "anti-CD4 antibody").
[5] The PD-1/CD4 bispecific antibody or antibody fragment thereof according to the preceding item [4], having a structure in which in order of the VL of anti-PD-1 antibody, the VH of anti-CD4 antibody, the VL of anti-CD4 antibody and the VH of anti-PD-1 antibody from its N-terminus, each is linked via peptide linker(s) or directly to each other.
[6] The PD-1/CD4 bispecific antibody or antibody fragment thereof according to the preceding item [4], having a structure in which in order of the VL of anti-PD-1 antibody, the VH of anti-PD-1 antibody, the VH of anti-CD4 antibody and the VL of anti-CD4 antibody from its N-terminus, each is linked via peptide linker(s) or directly to each other.
[7] The PD-1/CD4 bispecific antibody or antibody fragment thereof according to the preceding item [4], having a structure in which in order of the VL of anti-CD4 antibody, the VH of anti-PD-1 antibody, the VL of anti-PD-1 antibody and the VH of anti-CD4 antibody from its N-terminus, each is linked via peptide linker(s) or directly to each other.
[8] The PD-1/CD4 bispecific antibody or antibody fragment thereof according to any one of the preceding items [5] to [7], having a structure in which the N-terminus of a polypeptide having a human IgG constant region from a hinge site to a CH3 region (hereinafter, may be abbreviated as "IgG constant region (hinge/CH3)") is further linked via a peptide linker or directly to the C-terminus of the VH of anti-PD-1 antibody of the preceding item [5], that of the VL of anti-CD4 antibody of the preceding item [6] or that of the VH of anti-CD4 antibody of the preceding item [7].
[9] The PD-1/CD4 bispecific antibody or antibody fragment thereof according to the preceding item [4], having a structure in which in order of the VL of anti-CD4 antibody, the VH of anti-CD4 antibody, IgG constant region (hinge/CH3), the VH of anti-PD-1 antibody and the VL of anti-PD-1 antibody from its N-terminus, each is linked via peptide linker(s) or directly to each other.
[10] The PD-1/CD4 bispecific antibody or antibody fragment thereof according to the preceding item [8] or [9], further having a structure including a polypeptide having IgG constant region (hinge/CH3).
[11] The PD-1/CD4 bispecific antibody or antibody fragment thereof according to any one of the preceding items [5] to [10], wherein the peptide linker is Gly-Gly-Gly-Gly-Ser (SEQ ID NO: 1; herein "Gly" represents glycine, "Ser" represents serine and hereinafter, it may be abbreviated as "(Gly)×4-Ser)" or Gly-Gly-Gly-Gly-Ser-Gly-Gly-Gly-Gly-Ser-Gly-Gly-Gly-Gly-Ser (SEQ ID NO: 2; hereinafter, may be abbreviated as "((Gly)×4-Ser)×3") (herein, if there is a plurality of peptide linkers, each may be the same or different, and at least one of them may be that peptide linker).
[12] The PD-1/CD4 bispecific antibody or antibody fragment thereof according to any one of the preceding items [2] to [4], which is in a form of diabody, bispecific sc(Fv)₂, bispecific minibody, bispecific F(ab')₂, bispecific hybrid antibody, covalent diabody (bispecific DART), bispecific (FvCys)₂, bispecific F(ab'-zipper)₂, bispecific (Fv-zipper)₂, bispecific triple-chain antibody or bispecific mAb₂.
[13] The PD-1/CD4 bispecific antibody or antibody fragment thereof according to any one of the preceding items [2] to [4], which is in a form of bispecific hybrid antibody.
[14] The PD-1/CD4 bispecific antibody or antibody fragment thereof according to any one of the preceding items [2] to [4] and [13], which is an IgG antibody.
[15] The PD-1/CD4 bispecific antibody or antibody fragment thereof according to the preceding item [14], wherein the IgG antibody is an IgG₁ antibody or IgG₄ antibody.
[16] The PD-1/CD4 bispecific antibody or antibody fragment thereof according to the preceding item [15], wherein the binding of the IgG₁ antibody to Fc receptor is eliminated or decreased.
[17] The PD-1/CD4 bispecific antibody or antibody fragment thereof according to the preceding item [15] or [16], wherein the ADCC and/or CDC activity of the IgG₁ antibody is eliminated or decreased.
[18] The PD-1/CD4 bispecific antibody or antibody fragment thereof according to any one of the preceding items [8] to [10], wherein the isotype of the IgG constant region (hinge/CH3) is IgG₁.
[19] The PD-1/CD4 bispecific antibody or antibody fragment thereof according to any one of the preceding items [1] to [18], wherein PD-1 and CD4 are human PD-1 and human CD4, respectively.
[20] The PD-1/CD4 bispecific antibody or antibody fragment thereof according to any one of the preceding items [2] to [19], being a monoclonal antibody.
[21] The PD-1/CD4 bispecific antibody or antibody fragment thereof according to any one of the preceding items [2] to [20], being a humanized or fully human-type antibody.
[22] The PD-1/CD4 bispecific antibody or antibody fragment thereof according to any one of the preceding items [2] to [21], being an isolated antibody.
[23] The bispecific protein according to the preceding item [1], wherein the bispecific protein is a Scaffold molecule or modified peptide.
[24] The bispecific protein according to the preceding item [23], wherein the Scaffold molecule is in a form of Adnectin, Affibody (registered trademark), Anticalin (registered trademark), Avimer, DARPin, LRRP, Affilin (registered trademark), Affitin or Fynomer, and the modified peptide is in a form of a special cyclic peptide, Addbody (registered trademark) or Mirabody (registered trademark).
[25] The PD-1/CD4 bispecific protein or PD-1/CD4 bispecific antibody or antibody fragment thereof according to any one of the preceding items [1] to [24], which transmits (or being used to transmit) the inhibitory signal of PD-1.
[26] The PD-1/CD4 bispecific protein or PD-1/CD4 bispecific antibody or antibody fragment thereof according to any one of the preceding items [1] to [25], wherein the first arm allows the interaction with PD-1 and PD-L1, interaction with PD-1 and PD-L2, or both of interactions thereof (hereinafter, may be abbreviated as "allowing the interaction with PD-1 and PD-L").
[27] The PD-1/CD4 bispecific protein or PD-1/CD4 bispecific antibody or antibody fragment thereof according to any one of the preceding items [1] to [26], wherein the cytokine production or release during or within 24 hours after administration is sufficiently reduced.
[28] The PD-1/CD4 bispecific protein or PD-1/CD4 bispecific antibody or antibody fragment thereof according to any one of the preceding items [1] to [25], wherein the first arm allows the interaction with PD-1 and PD-L1 and the cytokine production or release during or within 24 hours after administration is sufficiently reduced.
[29] The PD-1/CD4 bispecific protein or PD-1/CD4 bispecific antibody or antibody fragment thereof according to the preceding item [27] or [28], wherein the cytokine is at least IL-2, IFN-γ, or TNF-α.
[30] The PD-1/CD4 bispecific protein or PD-1/CD4 bispecific antibody or antibody fragment thereof according to any one of the preceding items [1] to [29], which specifically binds to PD-1 and CD4 expressed on lymphocytes (e.g., helper T cells or regulatory T cells, etc.), respectively.
[31] The PD-1/CD4 bispecific protein or PD-1/CD4 bispecific antibody or antibody fragment thereof according to any one of the preceding items [1] to [29], which specifically binds to PD-1 expressed on cancer cells of hematological cancer as target cells, and CD4 expressed on lymphocytes as effector cells (e.g., helper T cells or regulatory T cells), respectively.
[32] The PD-1/CD4 bispecific protein or PD-1/CD4 bispecific antibody or antibody fragment thereof according to any one of the preceding items [1] to [29], which specifically binds to PD-1 and CD4 expressed on cancer cells derived from T cells as target cells, respectively.
[33] An isolated PD-1/CD4 bispecific antibody having a first arm specifically binding to PD-1 and a second arm specifically binding to CD4,
   (a) wherein the first arm contains a VH and VL of anti-PD-1 antibody, and the second arm contains a VH and VL of anti-CD4 antibody,
   (b) which specifically binds to PD-1 and CD4 expressed on lymphocytes (e.g., helper T cells or regulatory T cells, etc.), respectively, and
   (c) (i) which transmits the inhibitory signal of PD-1 and (ii) wherein the first arm allows the interaction with PD-1 and PD-L1, interaction with PD-1 and PD-L2, or both of interactions thereof, and/or (iii) wherein the cytokine production or release during or within 24 hours after administration is sufficiently reduced.
[34] An isolated humanized or fully human-type PD-1/CD4 bispecific antibody having a first arm specifically binding to PD-1 and a second arm specifically binding to CD4,
   (a) wherein the first arm contains a VH and VL of anti-human PD-1 antibody, and the second arm contains a VH and VL of anti-human CD4 antibody,
   (b) which specifically binds to PD-1 and CD4 expressed on lymphocytes (e.g., helper T cells or regulatory T cells, etc.), respectively,
   (c) (i) which transmits the inhibitory signal of PD-1 and (ii) wherein the first arm allows the interaction with PD-1 and PD-L1, and/or (iii) wherein the cytokine production or release during or within 24 hours after administration is sufficiently reduced, and
   (d) which is (i) in a form of bispecific hybrid antibody, and/or (ii) an IgG₁ antibody or IgG₄ antibody.
[35] An isolated PD-1/CD4 bispecific antibody having a first arm specifically binding to PD-1 and a second arm specifically binding to CD4,
   (a) wherein the first arm contains a VH and VL of anti-PD-1 antibody, and the second arm contains a VH and VL of anti-CD4 antibody,
   (b) (i) which specifically binds to PD-1 expressed on cancer cells of hematological cancer as target cells, and CD4 expressed on lymphocytes as effector cells (e.g., helper T cells or regulatory T cells), respectively, or (ii) which specifically binding to PD-1 and CD4 expressed on cancer cells derived from T cells as target cells, respectively, and
   (c) wherein the cytokine production or release during or within 24 hours after administration is sufficiently reduced.
[36] An isolated humanized or fully human-type PD-1/CD4 bispecific antibody having a first arm specifically binding to PD-1 and a second arm specifically binding to CD4,
   (a) wherein the first arm contains a VH and VL of anti-human PD-1 antibody, and the second arm contains a VH and VL of anti-human CD4 antibody,
   (b) (i) which specifically binds to PD-1 expressed on cancer cells of hematological cancer as target cells, and CD4 expressed on lymphocytes as effector cells (e.g., helper T cells or regulatory T cells), respectively, or (ii) which specifically binds to PD-1 and CD4 expressed on cancer cells derived from T cells as target cells, respectively,
   (c) wherein the cytokine production or release during or within 24 hours after administration is sufficiently reduced, and
   (d) which is (i) in a form of bispecific hybrid antibody, and/or (ii) an IgG₁ antibody or IgG₄ antibody.

[1-1] A pharmaceutical composition containing the PD-1/CD4 bispecific protein or PD-1/CD4 bispecific antibody or antibody fragment thereof selected from any one of the preceding items [1] to [36], as an active ingredient.

[1-2] The pharmaceutical composition according to the preceding item [1-1], which further contains a pharmaceutically acceptable carrier.

[2-1] An agent for preventing, suppressing the progression of symptoms of or the recurrence of and/or treating autoimmune disease, containing the PD-1/CD4 bispecific protein or PD-1/CD4 bispecific antibody or antibody fragment thereof selected from any one of the preceding items [1] to [30], [33] and [34] as an active ingredient.

[2-2] The agent according to the preceding item [2-1], wherein autoimmune disease is Behcet disease, systemic lupus erythematosus, chronic discoid lupus erythematosus, multiple sclerosis, systemic scleroderma, progressive systemic scleroderma, scleroderma, polymyositis, dermatomyositis, periarteritis nodosa (polyarteritis nodosa and microscopic polyangiitis), aortitis syndrome (Takayasu arteritis), malignant rheumatoid arthritis, rheumatoid arthritis, juvenile idiopathic arthritis, spondylarthritis, mixed connective tissue disease, Castleman Disease, Sjogren syndrome, adult-onset Still's disease, vasculitis, allergic granulomatous angiitis, hypersensitivity angiitis, rheumatoid vasculitis, large-vessel vasculitis, ANCA-associated vasculitis (e.g., granulomatosis with polyangiitis and eosinophilic granulomatosis with polyangiitis), Cogan's syndrome, RS3PE syndrome, temporal arteritis, giant-cell arteritis, polymyalgia rheumatica, fibromyalgia syndrome, antiphospholipid antibody syndrome, eosinophilic fasciitis, IgG4-related diseases (e.g., primary sclerosing cholangitis and autoimmune pancreatitis, etc.), Guillain-Barre syndrome, myasthenia gravis, chronic atrophic gastritis, autoimmune hepatitis, non-alcoholic steatohepatitis, primary biliary cirrhosis, Goodpasture's syndrome, rapidly progressive glomerulonephritis, lupus nephritis, megaloblastic anemia, autoimmune hemolytic anemia, pernicious anemia, autoimmune neutropenia, idiopathic thrombocytopenic purpura, Basedow's disease (Graves' disease (hyperthyroidism)), Hashimoto's thyroiditis, autoimmune adrenal insufficiency, primary hypothyroidism, Addison's disease (chronic adrenal insufficiency), idiopathic Addison's disease, type I diabetes mellitus, slowly progressive type I diabetes mellitus (latent autoimmune diabetes in adult), localized scleroderma, psoriasis, psoriatic arthritis, bullous pemphigoid, pemphigus, pemphigoid, herpes gestationis, linear IgA bullous skin disease, epidermolysis bullosa acquisita, alopecia areata, vitiligo, vitiligo vulgaris, neuromyelitis optica, chronic inflammatory demyelinating polyneuropathy, multifocal motor neuropathy, sarcoidosis, giant cell arteritis, amyotrophic lateral sclerosis, Harada disease, autoimmune optic neuropathy, idiopathic azoospermia, habitual abortion, inflammatory bowel disease (e.g., ulcerative colitis, Crohn's disease), celiac disease, ankylosing spondylitis, severe asthma, chronic urticaria, transplantation immunity, familial Mediterranean fever, eosinophilic sinusitis, dilated cardiomyopathy, systemic mastocytosis or inclusion body myositis.

[2-3] An agent for preventing, suppressing the progression of symptoms of or the recurrence of and/or treating autoimmune disease or graft-versus-host disease (GVHD), containing the PD-1/CD4 bispecific protein or PD-1/CD4 bispecific antibody or antibody fragment thereof selected from any one of the preceding items [1] to [30], [33] and [34] as an active ingredient.

[2-4] An agent for preventing, suppressing the progression of symptoms of or the recurrence of and/or treating type I diabetes mellitus, containing the PD-1/CD4 bispecific protein or PD-1/CD4 bispecific antibody or antibody fragment thereof selected from any one of the preceding items [1] to [30], [33] and [34] as an active ingredient, and being administered along with any one or more drugs selected from an insulin formulation (e.g., human insulin, insulin glargine, insulin lispro, insulin detemir and insulin aspart, etc.), sulfonylurea agent (e.g., Glibenclamide, Gliclazide and Glimepiride, etc.), quick-acting insulin secretion promoter (e.g., Nateglinide etc.), biguanide preparation (e.g., Metformin etc.), insulin resistance improving agent (e.g., Pioglitazone etc.), α-glucosidase inhibitor (e.g., Acarbose and Voglibose, etc.), diabetic neuropathy therapeutic agent (e.g., Epalrestat, Mexiletine and Imidapril, etc.), GLP-1 analog preparation (e.g., Liraglutide, Exenatide and Lixisenatide, etc.), and DPP-4 inhibitor (e.g., Sitagliptin, Vildagliptin and Alogliptin, etc.).

[2-5] An agent for preventing, suppressing the progression of symptoms of or the recurrence of, and/or treating multiple sclerosis, containing the PD-1/CD4 bispecific protein or PD-1/CD4 bispecific antibody or antibody fragment thereof selected from any one of the preceding items [1] to [30], [33] and [34] as an active ingredient, and being administered along with any one or more drugs selected from a steroid agent (e.g., cortisone, cortisone acetate, hydrocortisone, hydrocortisone sodium phosphate, hydrocortisone sodium succinate, fludrocortisone acetate, prednisolone, prednisolone acetate, prednisolone sodium succinate, prednisolone butylacetate, prednisolone sodium phosphate, halopredone acetate, methylprednisolone, methylprednisolone acetate, methylprednisolone sodium succinate, triamcinolone, triamcinolone acetate, triamcinolone acetonide, dexamethasone, dexamethasone acetate, dexamethasone valerate, dexamethasone cipecilate, dexamethasone palmitate, dexamethasone propionate, dexamethasone sodium phosphate, dexamethasone sodium metasulfobenzoate, paramethasone, paramethasone acetate, betamethasone, betamethasone dipropionate, betamethasone valerate, betamethasone acetate, betamethasone butyrate propionate and betamethasone sodium phosphate, etc.), interferon β-1a, interferon β-1b, glatiramer acetate, Mitoxantrone, Azathioprine, Cyclophosphamide, Ciclospolin, Methotrexate, Cladribine, adrenocorticotropic hormone (ACTH), Corticotropin, Mizoribine, Tacrolimus, Fingolimod, and Alemtuzumab.

[2-6] An agent for preventing, suppressing the progression of symptoms of or the recurrence of and/or treating systemic lupus erythematosus, containing the PD-1/CD4 bispecific protein or PD-1/CD4 bispecific antibody or antibody fragment thereof selected from any one of the preceding items [1] to [30], [33] and [34] as an active ingredient, and being administered along with any one or more drugs selected from a steroid agent (e.g., the steroid agents described in the preceding item [2-5]), immunosuppressive agent (e.g., Ciclospolin, Tacrolimus and Fingolimod, etc.) and Belimumab.

[2-7] An agent for preventing, suppressing the progression of symptoms of or the recurrence of and/or treating rheumatoid arthritis, containing the PD-1/CD4 bispecific protein or PD-1/CD4 bispecific antibody or antibody fragment thereof selected from any one of the preceding items [1] to [30], [33] and [34] as an active ingredient, and being administered along with any one or more drugs selected from a steroid agent (e.g., the steroid agents described in the preceding item [2-5]), anti-rheumatic agent (e.g., Methotrexate, Sulfasalazine, Bucillamine, Leflunomide, Mizoribine and Tacrolimus, etc.), anti-cytokine agent (e.g., Infliximab, Adalimumab, Tocilizumab, Etanercept, Golimumab and Certolizumab, etc.) and Abatacept.

[2-8] An agent for preventing, suppressing the progression of symptoms of or the recurrence of and/or treating autoimmune disease, containing the PD-1/CD4 bispecific protein or PD-1/CD4 bispecific antibody and the like selected from any one of the preceding items [1] to [30], [33] and [34] as an active ingredient, and being administered along with any one or more of drugs described in the preceding items [2-4] to [2-7].

[2-9] The agent for preventing, suppressing the progression of symptoms of or the recurrence of and/or treating each disease described in the preceding items [2-4] to [2-8], which is administered to the patient to which any one or more of drugs described in the preceding items [2-4] to [2-7] is/are administered.

[2-10] The agent for preventing, suppressing the progression of symptoms of or the recurrence of and/or treating each disease described in the preceding items [2-4] to [2-8], which is administered after administration of any one or more of drugs described in the preceding items [2-4] to [2-7].

[2-11] The agent for preventing, suppressing the progression of symptoms of or the recurrence of and/or treating each disease described in the preceding items [2-4] to [2-8], which is administered before administration of any one or more of drugs described in the preceding items [2-4] to [2-7].

[3-1] An agent for preventing, suppressing the progression of symptoms of or the recurrence of and/or treating hematological cancer, containing the PD-1/CD4 bispecific protein or PD-1/CD4 bispecific antibody or antibody fragment thereof selected from any one of the preceding items [1] to [29], [31], [32], [35] and [36] as an active ingredient.

[3-2] The agent according to the preceding item [3-1], wherein the hematological cancer is/are one or more cancers selected from multiple myeloma, malignant lymphoma, leukemia, primary central nervous system lymphoma and myeloproliferative syndrome.

[3-3] The agent according to the preceding item [3-2], wherein the hematological cancer is malignant lymphoma, further which is Non-Hodgkin's lymphoma or Hodgkin's lymphoma.

[3-4] The agent according to the preceding item [3-3], wherein malignant lymphoma is Non-Hodgkin's lymphoma, further which is B-cell non-Hodgkin's lymphoma or T/NK-cell non-Hodgkin's lymphoma.

[3-5] The agent according to the preceding item [3-4], wherein Non-Hodgkin's lymphoma is B-cell non-Hodgkin's lymphoma, further which is precursor B-cell lymphoblastic lymphoma, precursor B-cell acute lymphoblastic leukemia, chronic B-lymphoid leukemia, B-cell prolymphocytic leukemia, lymphoplasmacytic lymphoma, nodal marginal zone B-cell lymphoma, extranodal marginal zone B-cell lymphoma, primary splenic marginal zone B-cell lymphoma, hairy cell leukemia, hairly cell leukemia-variant, follicular lymphoma, pediatric type follicular lymphoma, diffuse large B cell lymphoma, diffuse large B-cell lymphoma, not otherwise specified, splenic diffuse red pulp small B-cell lymphoma, lymphoplasmacytic lymphoma, primary mediastinal large B-cell lymphoma, primary effusion lymphoma, Burkitt's lymphoma, mantle cell lymphoma, monoclonal B-cell lymphocytosis, splenic B-cell lymphoma/leukemia, unclassifiable, IgM monoclonal gammopathy of undetermined significance, µ heavy chain disease, λ heavy chain disease, α heavy chain disease, plasma cell myeloma, solitary plasmacytoma of bone, extraosseous plasmacytoma, monoclonal immunoglobulin deposition disease, large B-cell lymphoma with IRF4 rearrangement, primary cutaneous follicle center lymphoma, T-cell/histiocyte-rich large B-cell lymphoma, primary diffuse large B-cell lymphoma of the central nervous system, primary cutaneous diffuse large B-cell lymphoma, leg type, EBV positive diffuse large B-cell lymphoma, not otherwise specified, EBV positive mucocutaneous ulcer, diffuse large B-cell lymphoma associated with chronic inflammation, lymphomatoid granulomatosis, intravascular large B-cell lymphoma, ALK positive large B-cell lymphoma, plasmablastic lymphoma, primary effusion lymphoma, HHV8 positive diffuse large B-cell lymphoma, not otherwise specified, Burkitt-like lymphoma with 11q aberration, high-grade B-cell lymphoma, with MYC and BCL2 and/or BCL6 rearrangement, high-grade B-cell lymphoma, not otherwise specified or B-cell lymphoma, unclassifiable, with intermediate features between diffuse large B-cell lymphoma and classical Hodgkin lymphoma.

[3-6] The agent according to the preceding item [3-4], wherein Non-Hodgkin's lymphoma is T/NK-cell non-Hodgkin's lymphoma, further which is precursor T-cell lymphoblastic lymphoma, chronic T-cell lymphocytic leukemia, T-cell acute lymphocytic leukemia (T-cell lymphoblastic leukemia), T-cell large granular lymphoblastic leukemia, NK-cell large granular leukemia, aggressive NK-cell leukemia, peripheral T-cell lymphoma, peripheral T-cell lymphoma, not otherwise specified, unclassifiable peripheral T-cell lymphoma, angioimmunoblastic T-cell lymphoma, (CD30-positive) anaplastic large cell lymphoma, angiocentric lymphoma, intestinal T-cell lymphoma, enteropathy-type T-cell lymphoma, hepatosplenic gamma-delta T cell lymphoma, subcutaneous panniculitis-like T-cell lymphoma, mycosis fungoides, Sezary syndrome, Hodgkin-like/Hodgkin-related anaplastic large cell lymphoma, extranodal NK/T-cell lymphoma, adult T cell leukemia, T-cell prolymphocytic leukemia, chronic lymphoproliferative disorder of NK-cells, systemic EBV positive T-cell lymphoma of childhood, hydroa vacciniforme-like lymphoproliferative disorder, extranodal NK/T-cell lymphoma, nasal type, enteropathy-associated T-cell lymphoma, monomorphic epitheliotropic intestinal T-cell lymphoma, indolent T-cell lymphoproliferative disorder of the GI tract, hepatosplenic T-cell lymphoma, primary cutaneous CD30 positive T-cell lymphoproliferative disorders, lymphomatoid papulosis, primary cutaneous anaplastic large cell lymphoma, primary cutaneous gamma-delta T-cell lymphoma, primary cutaneous CD8 positive aggressive epidermotropic cytotoxic T-cell lymphoma, primary cutaneous acral CD8 positive T-cell lymphoma, primary cutaneous CD4 positive small/medium T-cell lymphoproliferative disorder, follicular T-cell lymphoma, nodal peripheral T-cell lymphoma with TFH phenotype, anaplastic large cell lymphoma, ALK positive, anaplastic large cell lymphoma, ALK negative or breast implant-associated anaplastic large-cell lymphoma.

[3-7] The agent according to the preceding item [3-3], wherein malignant lymphoma is Hodgkin's lymphoma, further which is classical Hodgkin's lymphoma (e.g., nodular sclerosis, mixed cellularity, lymphocyte-rich and lymphopenic) or nodular lymphocyte predominant Hodgkin's lymphoma.

[3-8] The agent according to the preceding item [3-2], wherein the hematological cancer is leukemia, further which is acute myelogenous leukemia, acute promyelocytic leukemia, acute lymphoblastic leukemia, chronic lymphocytic leukemia, myelodysplastic syndrome or chronic myelogenous leukemia.

[3-9] The agent according to any one of the preceding items [3-1] to [3-8], wherein the hematological cancer is pediatric hematological cancer.

[3-10] The agent according to any one of the preceding items [3-1] to [3-9], wherein the hematological cancer is the hematological cancer on which the therapeutic effect with other anti-cancer drugs is insufficient or is not sufficient.

[3-11] The agent according to any one of the preceding items [3-1] to [3-10], wherein the hematological cancer is exacerbated after treatment with other anti-cancer drugs.

[3-12] The agent according to any one of the preceding items [3-1] to [3-9], wherein a patient having hematologic cancer has no history of treatment with other anti-cancer drugs.

[3-13] The agent according to any one of the preceding items [3-1] to [3-12], wherein the hematological cancer is relapsed and/or refractory.

[3-14] The agent according to any one of the preceding items [3-1] to [3-13], which is further used in combination with other anti-cancer drugs.

[3-15] The agent according to the preceding item [3-10], [3-11] or [3-14], wherein the other anti-cancer drug is/are one or more drugs selected from an alkylating agent, platinum agent, antimetabolite (e.g., antifolate, pyridine metabolism inhibitor and purine metabolism inhibitor), ribonucleotide reductase inhibitor, nucleotide analog, topoisomerase inhibitor, microtubule polymerization inhibitor, microtubule depolymerization inhibitor, antitumor antibiotic, cytokine preparation, molecular targeting drug and cancer immunotherapeutic drug.

[3-16] The agent according to the preceding item [3-14] or [3-15], which is administered to a patient to which any one or more of the other anti-cancer drugs are administered.

[3-17] The agent according to the preceding item [3-14] or [3-15], which is administered after administration of any one or more of the other anti-cancer drugs.

[3-18] The agent according to the preceding item [3-14] or [3-15], which is administered prior to administration of any one or more of the other anti-cancer drugs.

[3-19] The agent according to any one of the preceding items [3-1] or [3-18], which is administered along with a steroid drug.

[3-20] The agent according to any one of the preceding items [3-1] to [3-18], which is administered after administration of a steroid drug.

[4-1] A hematological cancer cell growth inhibitor containing the PD-1/CD4 bispecific protein or PD-1/CD4 bispecific antibody or antibody fragment thereof selected from any one of the preceding items [1] to [29], [31], [32], [35] and [36] as an active ingredient.

[5-1] An intravenous injection formulation containing the PD-1/CD4 bispecific protein or PD-1/CD4 bispecific antibody or antibody fragment thereof selected from any one of the preceding items [1] to [36] and a pharmaceutically acceptable carrier.

[5-2] The intravenous injection formulation according to the preceding item [5-1] for preventing, suppressing the progression of symptoms of or the recurrence of and/or treating autoimmune disease, graft-versus-host disease (GVHD) or hematological cancer.

[5-3] The intravenous injection formulation according to the preceding item [5-1] or [5-2], being for drip infusion.

[6-1] A method for preventing, suppressing the progression of symptoms of or the recurrence of and/or treating autoimmune disease or graft-versus-host disease (GVHD), comprising administering an effective amount of the PD-1/CD4 bispecific protein or PD-1/CD4 bispecific antibody or antibody fragment thereof selected from any one of the preceding items [1] to [30], [33] and [34] to a patient.

[6-2] A method for preventing, suppressing the progression of symptoms of or the recurrence of and/or treating hematological cancer, comprising administering an effective amount of the PD-1/CD4 bispecific protein or PD-1/CD4 bispecific antibody or antibody fragment thereof selected from any one of the preceding items [1] to [29], [31], [32], [35] and [36] to a patient.

[6-3] A PD-1/CD4 bispecific protein or PD-1/CD4 bispecific antibody or antibody fragment thereof selected from any one of the preceding items [1] to [30], [33] and [34] for use in preventing, suppressing the progression of symptoms of or the recurrence of and/or treating autoimmune disease or graft-versus-host disease (GVHD).

[6-4] A PD-1/CD4 bispecific protein or PD-1/CD4 bispecific antibody or antibody fragment thereof selected from any one of the preceding items [1] to [29], [31], [32], [35] and [36] for use in preventing, suppressing the progression of symptoms of or the recurrence of and/or treating hematological cancer.

[6-5] Use of a PD-1/CD4 bispecific protein or PD-1/CD4 bispecific antibody or antibody fragment thereof selected from any one of the preceding items [1] to [30], [33] and [34] for manufacturing a pharmaceutical agent for preventing, suppressing the progression of symptoms of or the recurrence of and/or treating autoimmune disease or graft-versus-host disease (GVHD).

[6-6] Use of a PD-1/CD4 bispecific protein or PD-1/CD4 bispecific antibody or antibody fragment thereof selected from any one of the preceding items [1] to [29], [31], [32], [35] and [36] for manufacturing a pharmaceutical agent for preventing, suppressing the progression of symptoms of or the recurrence of and/or treating hematological cancer.

### [Advantage Effects of Invention]

The PD-1/CD4 bispecific protein of the present invention can be used for preventing, suppressing the progression of symptoms of or the recurrence of and/or treating autoimmune disease, hematological cancer and the like.

The PD-1/CD4 bispecific protein of the present invention can reduce the induction of cytokine production or release upon administration, and thus is expected to inhibit the development of infusion reactions or cytokine release syndrome after administration.

### [Brief Description of the Drawings]

[Figure 1] It shows a structure of the PD-1/CD4 bispecific antibody described in Example 1 (hereinafter, may be abbreviated as a "PD-1/CD4 scDb"). In the figure, the "VH CD4" and "VL CD4" represent a VH and VL of anti-CD4 antibody, respectively, and the "VH PD-1" and "VL PD-1" represent a VH and VL of anti-PD-1 antibody, respectively. The "L1", "L2" and "L3" represent a peptide linker linking the respective VHs and VLs, and the L1 and L3 have a structure represented by (Gly)×4-Ser and the L2 has a structure represented by ((Gly)×4-Ser)×3.
[Figure 2] It shows a structure of the PD-1/CD4 bispecific antibody described in Example 2(hereinafter, may be abbreviated as a "PD-1/CD4 scDb-Fc"). In the figure, the "IgG₁(Hinge-CH3)" represents a human IgG₁ constant region having a hinge site to a CH3 region. The "L4" represents a peptide having a structure represented by (Gly)×4-Ser or ((Gly)×4-Ser)×3. Other symbols have the same meaning as shown in Figure 1.
[Figure 3] It shows flow cytometry showing specific binding properties of the PD-1/CD4 scDb to mouse PD-1. In the figure, Clone-1 and Clone-A to -H represent the PD-1/CD4 scDb prepared in Example 1, respectively.
[Figure 4] It shows flow cytometry showing specific binding properties of the PD-1/CD4 scDb-Fc to mouse PD-1. In the figure, Clone-2 and Clone-I to -P represent the PD-1/CD4 scDb-Fc prepared in Example 2, respectively.
[Figure 5] It shows flow cytometry showing specific binding properties of the PD-1/CD4 scDb to mouse CD4.
[Figure 6] It shows flow cytometry showing specific binding properties of the PD-1/CD4 scDb-Fc to mouse CD4.
[Figure 7] It shows the effects of both PD-1/CD4 bispecific proteins (Clone-1 and Clone-2) in the experimental allergic encephalomyelitis mouse model (EAE model).
[Figure 8] It shows the plasma cytokine levels in mice administered with the PD-1/CD4 bispecific protein. In the figure, "∗" and "∗∗" indicate significance at p<0.05 and p<0.01, respectively, by t-test with Vehicle.

### [Description of Embodiments]

An embodiment of PD-1 (Programmed Cell Death-1) is a membrane-type protein comprising the amino acid sequence represented by GenBank accession number NP_005009. When described as the term "PD-1" in the present specification, unless specifically defined otherwise, it may be used as a meaning including all of isoforms thereof and further variants thereof in which an epitope of the "first arm specifically binding to PD-1" of the present invention has been conserved. In the present invention, PD-1 is preferably human PD-1.

An embodiment of CD4 has the amino acid sequence represented by GenBank accession number NP_000607. When described as the term "CD4" in the present specification, unless specifically defined otherwise, it may be used as a meaning including all of isoforms thereof and further variants thereof in which an epitope of the "second arm specifically binding to CD4" of the present invention has been conserved. In the present invention, CD4 is preferably human CD4.

In the present specification, the term "isolate" means becoming a single substantially pure component by being identified, separated and/or purified from impurities containing a plurality of or myriad number of components extracted from host cells.

In the present specification, the term "bispecific protein" means a protein having the binding specificity to two different antigen molecules or epitopes in one molecule, and as long as it has the binding specificity, it may be in any form, including, for example, a bispecific antibody and an antibody fragment thereof, Scaffold molecule (see Current Opinion in Biotechnology (2006), Vol. 17, No.6, p.653-658, Current Opinion in Biotechnology (2007), Vol. 18, p.1-10, Current Opinion in Structural Biology (1997), Vol. 7, p. 463-469 or Protein Science (2006), Vol. 15, p. 14-27) and modified peptides (e.g.,, special cyclic peptides (J. Synth. Org. Chem., Jpn. (2017), Vol.75, No.11, p.1171-1178), Addbody (registered trademark) and Mirabody (registered trademark), etc.) and the like, but it is not limited to those. Further, examples of such Scaffold molecules include Adnectin (see WO2001/64942), Affibody (registered trademark) (see WO95/19374 and WO2000/63243), Anticalin (registered trademark) (see WO99/16873), Avimer (see Nature Biotechnology (2005), Vol. 23, p. 1556-1561), DARPin (see Nature Biotechnology (2004), Vol. 22, p.575-582), LRRP (see Nature (2004), Vol. 430, No. 6996, p.174-180), Affilin (Registered trademark) (see WO2001/04144 and WO2004/106368), Affitin (see Journal of molecular biology (2008), Vol. 383, No.5, p.1058-1068), Fynomer (WO2011/023685) and the like, but it is not limited to those.

In the present specification, the term "monoclonal antibody" means an antibody obtained from a substantially homogeneous antibody group binding to the same specific antigen.

In the present specification, the term "bispecific antibody" means an antibody having the binding specificity to two different antigen molecules or epitopes in one molecule. Further, the term "bispecific monoclonal antibody" means a bispecific antibody obtained from a substantially homogeneous antibody group.

Examples of forms of the bispecific antibodies include a diabody, bispecific sc(Fv)₂, bispecific minibody, bispecific F(ab')₂, bispecific hybrid antibody, covalent diabody (bispecific DART), bispecific (FvCys)₂, bispecific F(ab'-zipper)₂, bispecific (Fv-zipper)₂, bispecific three-chain antibody, bispecific mAb² and the like.

The diabody is a dimer of single-chain peptides in which a VH and VL recognizing different antigens were linked to each other with a peptide linker (see Proc. Natl. Acad. Sci. USA, 1993, Vol. 90, No. 14, pp. 6444-6448).

The bispecific sc(Fv)₂ is a low-molecular antibody modified such that two pairs of VH/VL of two antibodies recognizing different antigens are linked with a peptide linker to form a continuous single chain (see J. Biological Chemistry, 1994, 269: pp. 199-206).

The bispecific F(ab')₂ is a low-molecular antibody in which Fab' fragments of antibodies recognizing two different antigens were covalently bound through a disulfide bond or the like.

The bispecific minibody is a low-molecular antibody in which the low-molecular antibody fragments modified in such a manner that the constant region CH3 domains of the antibodies are linked to scFv recognizing different antigens, respectively, was covalently bonded by disulfide bonds or the like on the CH3 domains (see Biochemistry, 1992, Vo. 31, No .6, pp. 1579-1584).

The bispecific hybrid antibody is an intact antibody in which the heavy chain/light chain complexes recognizing two different antigens were covalently bound each other by disulfide bonds or the like.

In the present invention, the form of the bispecific antibody is preferably in a form with a wide movable area between two variable areas, for example, a bispecific hybrid antibody.

The bispecific hybrid antibody can be produced from a hybridoma produced by, for example, hybrid hybridoma method (see US4474893). Alternatively, the bispecific hybrid antibody can be produced by having mammal animal cells co-express four kinds of cDNAs encoding a heavy chain and light chain of antibody recognizing different antigens, respectively, and secrete it.

The monoclonal antibodies used in the present invention can be produced by hybridoma method (see, e.g., Kohler and Milstein et al., Nature (1975), Vol. 256, p.495-97, Hongo et al., Hybridoma (1995), Vol. 14, No.3, pp.253-260, Harlow et al., Antibodies: A Laboratory Manual, (Cold Spring Harbor Laboratory Press (1988), Vol. 2) and Hammerling et al., Monoclonal Antibodies and T-Cell Hybridomas, pp.563-681 (Elsevier, N.Y., 1981)), recombinant DNA method (see, e.g., US4816567), phage display method (see, e.g., Ladner et al., US5223409, US5403484 and US5571698, Dower et al., US5427908 and US5580717, McCafferty et al., US5969108 and US6172197, and Griffiths et al., US5885793, US6521404, US6544731, US6555313, US6582915 and US6593081).

An antibody or monoclonal antibody, when being administered to human, can be produced in a form of a chimeric antibody, humanized antibody or complete human antibody in order to reduce or eliminate its antigenicity.

The term "chimeric antibody" means an antibody of which the variable region sequence and constant region sequence are derived from different mammalian. Examples thereof include an antibody of which the variable region sequence is derived from a mouse antibody and the constant region sequence is derived from a human antibody. The chimeric antibody can be produced by linking a gene encoding an antibody variable region isolated from antibody-producing hybridomas isolated by the above-mentioned hybridoma method, recombinant DNA method or phage display method, by well-known techniques, to a gene encoding the constant region of human-derived antibody using well-known methods (see, e.g., Cabilly et al., US4816567).

The term "humanized antibody" means an antibody of which complementarity determining region (CDR) sequences derived from a germ line of other mammals such as mice were grafted into human framework sequences. The humanized antibody can also be produced by linking genes encoding the CDRs of antibody isolated from antibody-producing hybridomas isolated by the above-mentioned method, by well-known techniques, to a gene encoding a framework region of the human-derived antibody using well-known methods (see, e.g., Winter, US5225539 and US5530101; Queen et al., US5585089 and US6180370).

The term "human antibody" or "complete human antibody" means an antibody in which both of variable regions composed of framework regions and CDRs and constant regions are derived from human germline immunoglobulin sequences. The human antibody to be used in the present invention can be produced by a method using mice transformed to produce a human antibody, for example, Humab mice (see, e.g., Lonberg and Kay et al., US5545806, US5569825, US5625126, US5633425, US5789650, US5877397, US5661016, US5814318, US5874299 and US5770429), KM mice (see, e.g., Ishida et al., WO2002/43478), Xeno mice (see, e.g., US5939598, US6075181, US6114598, US6150584 and US6162963), or Tc mice (see, e.g., Tomizuka et al., Proc. Natl. Acad. Sci. USA (2000), pp.722-727). Alternatively, the human antibody can also be prepared using SCID mice in which human immune cells have been reconstructed such that the human antibody response is made upon immunization (see, e.g., Wilson et al., US5476996 and US5698767). Furthermore, the human antibody to be used in the present invention can also be produced according to the above-mentioned phage display method.

In the present specification, the term "antibody fragment" of the PD-1/CD4 bispecific antibody is a part of the full-length antibody, and is an antibody having an antigen binding part to PD-1 and an antigen binding part to CD4. Examples thereof include F(ab')₂, and the like. Herein, the antigen binding part means a minimum unit of an antibody which can bind to an antigen thereof, for example, it is composed of three CDRs in the respective VH and VL and framework regions to arrange CDRs such that the target antigen can be recognized by combination of those CDRs.

In the present specification, the term "isotype" means the antibody class (e.g., IgM or IgG) which is encoded by heavy chain constant region genes. The isotype for the bispecific antibody of the present invention is preferably IgG, and more preferably, IgG₁ or IgG₄. An embodiment of IgG₁ is of which the binding to Fc receptor (specifically, Fc-gamma receptor) was eliminated or decreased, or the ADCC and/or CDC activity was eliminated or decreased. Specifically, the IgG₁ antibody of which the binding to Fc receptor was eliminated or decreased can be obtained by substituting, deleting or inserting arbitrary amino acids of heavy chain constant region thereof. Examples thereof include an antibody in which each leucine at position 234 according to the EU numbering system was substituted with alanine and/or each leucine at position 235 was substituted with alanine on each of two heavy chain constant regions or hinge regions thereof. Examples thereof also include an antibody in which each leucine at position 235 according to the EU numbering system was substituted with glycine and/or each glycine at position 236 was substituted with arginine on each of two heavy chain constant regions or hinge regions of the bispecific antibody. Furthermore, in order to reduce the heterogeneity of antibody, an antibody in which an amino acid at the C-terminus, for example, lysine at position 447 according to the EU numbering system has been deleted is preferable.

In the Fc regions of the bispecific antibody of the present invention, arbitrary amino acids therein may be substituted such that two different heavy chains are easily associated with each other. Examples of preferable embodiments thereof include a PD-1/CD4 bispecific antibody of which in the constant region of the heavy chain having the VH of the first arm specifically binding to PD-1, leucine at position 351 according to the EU numbering system was substituted with lysine, and threonine at position 366 was substituted with lysine, and of which in the constant region of the heavy chain having the VH of the second arm specifically binding to CD4, leucine at position 351 was substituted with aspartic acid, and leucine at position 368 was substituted with glutamic acid. Furthermore, examples thereof also include a PD-1/CD4 bispecific antibody of which in the constant region in the heavy chain having the VH of the first arm specifically binding to PD-1, leucine at position 351 according to the EU numbering system was substituted with aspartic acid, and leucine at position 368 was substituted with glutamic acid, and of which in the constant region in the heavy chain having the VH of the second arm specifically binding to CD4, leucine at position 351 was substituted with lysine, and threonine at position 366 was substituted with lysine.

Furthermore, if the bispecific antibody of the present invention is of IgG₄, in order to suppress the swapping in an antibody molecule, a variant in which arbitrary amino acids in the heavy chain constant region thereof were substituted, deleted or inserted is preferable. Preferable examples thereof include an antibody of which serine at position 228 according to the EU numbering system, located in the hinge region thereof, was substituted with proline. Note here that in the present specification, amino acid positions assigned to CDRs and frameworks in variable regions of antibody may be specified according to Kabat's numbering system (see Sequences of Proteins of Immunological Interest (National Institute of Health, Bethesda, Md., 1987 and 1991)). Furthermore, amino acids in the constant region are indicated according to the EU numbering system based on Kabat's amino acid positions (see Sequences of proteins of immunological interest, NIH Publication No. 91-3242).

In the present document, the term "peptide linker" is not particularly limited as long as it can link the two sets of VH and VL comprising the bispecific antibody or antibody fragment thereof of the present invention and the IgG1 constant region (hinge/CH3) thereto, and does not inhibit the function and formation of the same bispecific antibody or antibody fragment thereof, and examples thereof include Ser, (Gly)ₙ-Ser, Ser-(Gly)n, ((Gly)₄-Ser)ₙ, (Ser-(Gly)₄)ₙ [n represents an integer of 1 to 6, and other symbols have the same meaning as above], and the like, and (Gly)x4-Ser and ((Gly)x4-Ser)x3 therein and the like are preferable. If the bispecific antibody or antibody fragment thereof has a plurality of peptide linkers, each may consist of the same or different amino acid sequence as described above, and at least one of them may be a peptide linker consisting of the amino acid sequence described above.

### The first arm specifically binding to PD-1

In the present specification, the "first arm specifically binding to PD-1" (hereinafter, may be abbreviated as the "first arm") means a protein specifically binding to PD-1, regardless of whether it is contained in a part of the bispecific protein of the present invention, or exists as a simple substance, and if the bispecific protein is a bispecific antibody, the first arm means a part of antibody containing at least a VH and VL of antibody specifically binding to PD-1 (hereinafter, may be abbreviated as an "anti-PD-1 antibody") and capable of specifically binding to PD-1, regardless of whether it is contained in a part of antibody or antibody fragment thereof, or exists not as a part but as a simple substance. For example, the first arm like this includes a Fv of the antibody comprising the VH and VL composing antigen binding parts of the anti-PD-1 antibody, as well as a Fab' and Fab of the antibody containing the same VH and VL. Herein, the term "specifically binding to PD-1" is used as a feature of directly binding to PD-1 with higher binding affinity than at least 1 × 10⁻⁵ M, preferably 1 × 10⁻⁷ M, and more preferably 1 × 10⁻⁹ M (dissociation constant (Kd value)), and not substantially binding to any receptor members belonging to a so-called CD28 family receptor, such as at least, CD28, CTLA-4 and ICOS. Furthermore, the "antibody" in the "antibody specifically binding to PD-1" or the "anti-PD-1 antibody" means a full-length antibody, that is, a full-length antibody consisting of two heavy chains and two light chains linked with disulfide bonds, and preferably a monoclonal antibody thereof.

An embodiment of the "first arm specifically binding to PD-1" allows the interaction between PD-1 and PD-L1, interaction between PD-1 and PD-L2 or both of interactions thereof. Herein, the sentence "allows the interaction between PD-1 and PD-L1, interaction between PD-1 and PD-L2, or both of interactions thereof" means that even when there is the PD-1/CD4 bispecific antibody of the present invention at 20-fold excess over the concentration of the soluble form of PD-L1 or PD-L2, the interaction between PD-L1 and PD-1, interaction between PD-L2 and PD-1, or both of interactions thereof are maintained 50% or more, preferably 70% or more, and more preferably 80% or more, compared with those when there is no PD-1/CD4 bispecific antibody of the present invention. Whether the first arm allows these interactions can be determined by measuring the effect of the first arm on the binding of soluble PD-1 protein to immobilized PD-L1 protein or PD-L1-expressing cells, using known methods, for example, biacore analysis, ELISA assay, flow cytometry, enzyme-linked immunosorbent assay (ELISA), fluorescence energy transfer assay (FRET) or fluorescence microanalysis technique (FMAT (registered trademark)). Furthermore, the definition of "allowing the interaction between PD-1 and PD-L1, interaction between PD-1 and PD-L2, or both of interactions thereof" may have the same meaning as that of "which does not substantially inhibit the interaction between PD-1 and PD-L1, interaction between PD-1 and PD-L2 or both of interactions thereof."

Another embodiment of the "first arm specifically binding to PD-1" of the present invention may include one which inhibits the interaction between PD-1 and PD-L1, interaction between PD-1 and PD-L2, or both of interactions thereof.

Furthermore, other examples of "the first arm specifically binding to PD-1" also include the combination of the VH and VL of a known anti-PD-1 antibody, and examples thereof include Nivolumab, Cemiplimab, Pembrolizumab, Spartalizumab, Tislelizumab, Dostarlimab, Toripalimab, Camrelizumab, Genolimzumab, Sintilimab, Lodapolimab, Retifanlimab, Balstilimab, Serplulimab, Budigalimab, Prolgolimab, Sasanlimab, Cetrelimab, Zimberelimab, Penpulimab, AMP-514(MEDI0680), STI-A1110, ENUM 388D4, ENUM 244C8, GLS010, CS1003, BAT-1306, AK103, BI 754091, LZM009, CMAB819, Sym021, SSI-361, JY034, HX008, ISU106, CX-188 and the like. Examples of the first arm specifically binding to PD-1 also include the combination of the VH and VL of the anti-PD-1 antibody disclosed in the patent publication identified by US20180222982, US20180312564, WO2004056875, WO2006121168, WO2008156712, WO2010029434, WO2010029435, WO2010036959, WO2011110604, WO2011110621, WO2014179664, WO2014194302, WO2014206107, WO2015035606, WO2015036394, WO2015085847, WO2015112800, WO2016014688, WO2016015685, WO2016068801, WO2016077397, WO2016092419, WO2016106159, WO2016127179, WO2016197497, WO2016210129, WO2017011580, WO2017016497, WO2017019846, WO2017024465, WO2017024515, WO2017025016, WO2017025051, WO2017040790, WO2017055443, WO2017055547, WO2017058115, WO2017058859, WO2017079112, WO2017079116, WO2017087599, WO2017096026, WO2017106656, WO2017107885, WO2017124050, WO2017125815, WO2017132827, WO2017133540, WO2017166804, WO2017198741, WO2017201766, WO2017214182, WO2018020476, WO2018026248, WO2018034226, WO2018036472, WO2018052818, WO2018053106, WO2018068336, WO2018085358, WO2018085468, WO2018087143, WO2018091661, WO2018113258, WO2018119474, WO2018162944, WO2018192089, WO2018210230, WO2018217227, WO2018226580, WO2019005635 or WO2019051164, respectively. Furthermore, examples of the first arm specifically binding to PD-1 also include the combination of the VH of the above-mentioned the anti-PD-1 antibody and the common light chain.

### The second arm specifically binding to CD4

In the present specification, the "second arm specifically binding to CD4" (hereinafter, may be abbreviated as the "second arm") means a protein specifically binding to CD4, regardless of whether it is contained in a part of the bispecific protein of the present invention, or exists as a simple substance, and if the bispecific protein is a bispecific antibody, the second arm means an antibody portion having at least a VH and VL of an antibody specifically binding to CD4 (hereinafter, may be abbreviated as an "anti-CD4 antibody") and capable of specifically binding to CD4, regardless of whether it is contained in a part of antibody or antibody fragment thereof, or exists not as a part but as a simple substance. For example, the second arm includes a Fv of the antibody comprising the VH and VL composing antigen binding parts of the anti-CD4 antibody, as well as a Fab' and Fab of the antibody containing the same VH and VL. Herein, the sentence "specifically binding to CD4" is used as a feature of directly binding to CD4 with higher binding affinity than at least 1 × 10⁻⁵ M, preferably 1 × 10⁻⁷ M, and more preferably 1 × 10⁻⁹ M (dissociation constant (Kd value)) and not substantially binding to any other proteins. Furthermore, the "antibody" in the "antibody specifically binding to CD4" or "anti-CD4 antibody" means a full-length antibody, that is, a full-length antibody consisting of two heavy chains and two light chains linked with disulfide bonds, and preferably a monoclonal antibody thereof.

Further, other examples of the "second arms specifically binding to CD4" include a combination of the VH and VL of a known anti-CD4 antibody, and examples thereof include MTRX-1011A, TRX-1, Ibalizumab, BT-061, huB-F5, Zanolimumab, 4162W94, Clenoliximab, Keliximab, AD-519, PRO-542, Cedelizumab, IT1208, and the like.

An isotype of the PD-1/CD4 bispecific antibody of the present invention is preferably an IgG antibody, further preferably an IgG₁ antibody or IgG₄ antibody.

In the bispecific antibody of the present invention, if the IgG₁ antibody, it may be in a form in which mutations substantially eliminating the binding to Fc receptor have been introduced, and examples thereof include an IgG₁ antibody in which each leucine at position 234 according to the EU numbering system was substituted with alanine and/or each leucine at position 235 was substituted with alanine on two heavy chain constant regions or hinge regions thereof. Alternatively, it may be an IgG₁ antibody in which each leucine at position 235 according to the EU numbering system was substituted with glycine, and/or each glycine at position 236 was substituted with arginine on two heavy chain constant regions or hinge regions thereof. Furthermore, the bispecific antibody in which the C-terminal amino acids of heavy chains, for example, lysine at position 447 according to the EU numbering system have been deleted is more preferable.

Furthermore, if these PD-1/CD4 bispecific antibodies are the IgG₁ antibodies, preferable embodiments thereof include those in which in the constant region of the heavy chain having the VH of the first arm specifically binding to PD-1, leucine at position 351 according to the EU numbering system was substituted with lysine and threonine at position 366 was substituted with lysine, and in the constant region of the heavy chain having the VH of the second arm specifically binding to CD4, leucine at position 351 was substituted with aspartic acid and leucine at position 368 was substituted with glutamic acid. Furthermore, the IgG₁ antibody in which in the constant region of the heavy chain having the VH of the first arm specifically binding to PD-1, leucine at position 351 according to the EU numbering system was substituted with aspartic acid and leucine at position 368 was substituted with glutamic acid, and in the constant region of the heavy chain having the VH of the second arm specifically binding to CD4, leucine at position 351 was substituted with lysine and threonine at position 366 was substituted with lysine is also preferable, as well.

On the other hand, if the PD-1/CD4 bispecific antibody is the IgG₄ antibody, an antibody in which serine at position 228 according to the EU numbering system, located in the hinge region thereof, was substituted with proline is preferable.

An embodiment of the PD-1/CD4 bispecific protein of the present invention (preferably a PD-1/CD4 bispecific antibody etc.) has the feature of transmitting the inhibitory signal of PD-1.

An embodiment of the PD-1/CD4 bispecific protein of the present invention (preferably a PD-1/CD4 bispecific antibody etc.) has the feature of allowing the interaction between PD-1 and PD-L1, interaction between PD-1 and PD-L2 or both of interactions thereof.

The PD-1/CD4 bispecific protein of the present invention (preferably a PD-1/CD4 bispecific antibody etc.) has the feature of sufficiently reducing cytokine production or release during or by 24 hours after administration. Herein, the sentence "sufficiently reducing cytokine production or release during or by 24 hours after administration" means that, for example, during intravenous administration or by 24 hours after that administration, by drip infusion of the PD-1/CD4 bispecific antibody of the present invention, for example, the concentration in blood or tissue of cytokine including IL-2, IFN-γ and/or TNF-α does not increase or even if it increases, it is such a degree that it can be suppressed by steroid administration.

An embodiment of the PD-1/CD4 bispecific protein of the present invention (preferably, a PD-1/CD4 bispecific antibody etc.) may specifically bind to PD-1 and CD4 expressed on lymphocytes (preferably, T-cell lymphocytes (e.g., helper T cells or regulatory T cells, etc.), etc.), respectively. In this case, it may bind to PD-1 and CD4 simultaneously, or it may bind to PD-1 and CD4 expressed on the same lymphocyte.

An embodiment of the PD-1/CD4 bispecific protein of the present invention (preferably, a PD-1/CD4 bispecific antibody, etc.) may specifically bind to PD-1 expressed on cancer cells of hematological cancer, which are target cells, and CD4 expressed on lymphocytes, which are effector cells, (preferably, T-cell lymphocytes (e.g., helper T cells or regulatory T cells, etc.) etc.), respectively, and on this case, it may bind to PD-1 and CD4, simultaneously. Herein, the term "target cell" means cells subject to cytotoxic effects of lymphocytes which are effector cells, and the term "effector cell" means cells giving cytotoxic effects thereof to target cells.

An embodiment of the PD-1/CD4 bispecific protein of the present invention (preferably, a PD-1/CD4 bispecific antibody etc.) may specifically bind to PD-1 and CD4 expressed on T-cell-derived cancer cells, respectively, in this case, it may bind to PD-1 and CD4, simultaneously.

In the present invention, the PD-1/CD4 bispecific protein is preferably a PD-1/CD4 bispecific antibody and an antibody fragments thereof, and the PD-1/CD4 bispecific antibody is preferably a PD-1/CD4 bispecific monoclonal antibody, more preferably an isolated PD-1/CD4 bispecific monoclonal antibody, furthermore preferably, an isolated human PD-1/human CD4 bispecific monoclonal antibody. Herein, the term "isolated human PD-1/human CD4 bispecific monoclonal antibody" means an isolated bispecific monoclonal antibody to human PD-1 and human CD4.

### Method for manufacturing and purifying the PD-1/CD4 bispecific antibody

The PD-1/CD4 bispecific antibody and antibody fragment thereof of the present invention can also be manufactured by the method disclosed in WO2014/051433, WO2013/157953 or WO2013/157954.

Specifically, it can be manufactured by gene-transferring an expression vector in which (1) a polynucleotide encoding the heavy chain having the VH of the anti-PD-1 antibody, (2) a polynucleotide encoding the heavy chain having the VH of the anti-CD4 antibody, (3) a polynucleotide encoding the light chain having the VL of the anti-PD-1 antibody, and (4) a polynucleotide encoding the light chain having the VL of the anti-CD4 antibody have been inserted, respectively, into mammalian animal cells to transform them, and then by having them express and secret both of the heavy chain and the light chain.

Herein, any host cells for expressing the bispecific antibody of the present invention can be used as long as they can be gene-introduced by expression vectors to express them. Preferable examples of host cells include insect cells such as SF-9 and SF-21, more preferably, mammalian cells such as mouse cells including CHO cells, BHK cells, SP2/0 cells and NS-0 myeloma cells, primate cells such as COS and Vero cells and MDCK cells, BRL 3A cells, hybridoma, tumor cells, immortalized primary cells, W138, HepG2, HeLa, HEK293, HT1080 and embryonic retina cells such as PER.C6, and the like. Note here that in selection of the expression system, expression vectors for mammalian cells and host cells therefor can often be used such that antibodies are appropriately glycosylated. Human cell lines, preferably, PER.C6 are advantageously used to obtain antibodies corresponding to glycosylated patterns for human.

Protein production in host cells transformed by gene-transferring the expression vectors can be carried out with reference to, for example, Current Protocols in Protein Science (1995), Coligan JE, Dunn BM, Ploegh HL, Speicher DW, Wingfield PT, ISBN 0-471-11184-8, Bendig, 1988. Further, general guidelines, procedures and practical methods to maximize the productivity of host cell culture can be carried out with reference to Mammalian Cell Biotechnology: a Practical Approach (M. Butler, ed., IRL Press, 1991). Expression of antibodies in host cells is described in, for example, publications such as EP0120694, EP0314161, EP0481790, EP0523949, US4816567, WO2000/63403 and the like.

Herein, culture conditions for host cells can be optimized by well-known methods, and the amount of protein production therein can be optimized. The culture can be carried out by batch culture, feeding culture, continuous culture or hollow-fiber culture in a petri dish, roller bottle or reaction chamber. In order to produce recombinant protein by cell culture in a large-scale and continuously, it is preferable to allow cells to proliferate in suspension. Furthermore, it is preferable to culture cells under a condition without any animal- or human-derived serum or animal- or human-derived serum components.

Antibodies expressed in host cells and recovered from them or culture thereof by well-known methods can be purified using well-known methods. Examples of purification method include immunoprecipitation method, centrifugation method, filtration, size-exclusion chromatography, affinity chromatography, cation and/or anion exchange chromatography, hydrophobic interaction chromatography and the like. Furthermore, protein A or protein G affinity chromatography may be preferably used (see, e.g., US4801687 and US5151504).

### [Pharmaceutical use]

The PD-1/CD4 bispecific protein of the present invention is useful for preventing, suppressing the progression of symptoms of or the recurrence of and/or treating autoimmune disease, graft-versus-host disease (GVHD) or hematological cancer.

Examples of autoimmune diseases in the present invention include Behcet disease, systemic lupus erythematosus, chronic discoid lupus erythematosus, multiple sclerosis, systemic scleroderma, progressive systemic scleroderma, scleroderma, polymyositis, dermatomyositis, periarteritis nodosa (polyarteritis nodosa and microscopic polyangiitis), aortitis syndrome (Takayasu arteritis), malignant rheumatoid arthritis, rheumatoid arthritis, juvenile idiopathic arthritis, spondylarthritis, mixed connective tissue disease, Castleman Disease, Sjogren syndrome, adult-onset Still's disease, vasculitis, allergic granulomatous angiitis, hypersensitivity angiitis, rheumatoid vasculitis, large-vessel vasculitis, ANCA-associated vasculitis (e.g., granulomatosis with polyangiitis and eosinophilic granulomatosis with polyangiitis), Cogan's syndrome, RS3PE syndrome, temporal arteritis, giant-cell arteritis, polymyalgia rheumatica, fibromyalgia syndrome, antiphospholipid antibody syndrome, eosinophilic fasciitis, IgG4-related diseases (e.g., primary sclerosing cholangitis and autoimmune pancreatitis, etc.), Guillain-Barre syndrome, myasthenia gravis, chronic atrophic gastritis, autoimmune hepatitis, non-alcoholic steatohepatitis, primary biliary cirrhosis, aortitis syndrome, Goodpasture's syndrome, rapidly progressive glomerulonephritis, lupus nephritis, anti-glomerular basement membrane nephritis, megaloblastic anemia, autoimmune hemolytic anemia, pernicious anemia, autoimmune neutropenia, idiopathic thrombocytopenic purpura, hyperthyroidism (Graves' disease (Basedow's disease)), Hashimoto's thyroiditis, autoimmune adrenal insufficiency, primary hypothyroidism, Addison's disease, idiopathic Addison's disease (chronic adrenal insufficiency), type I diabetes mellitus, slowly progressive type I diabetes mellitus, localized scleroderma, psoriasis, psoriatic arthritis, bullous pemphigoid, pemphigus, pemphigoid, herpes gestationis, linear IgA bullous skin disease, epidermolysis bullosa acquisita, alopecia areata, vitiligo, vitiligo vulgaris, Harada disease, autoimmune optic neuropathy, idiopathic azoospermia, habitual abortion, inflammatory bowel disease (e.g., ulcerative colitis, Crohn's disease), celiac disease, atopic dermatitis, neuromyelitis optica, chronic inflammatory demyelinating polyneuropathy, multifocal motor neuropathy, pneumocystinosis, autoimmune hemorrhagic disease XIII, remittent polychondritis, sarcoidosis, , ankylosing spondylitis, severe asthma, chronic urticaria, transplantation immunity, familial Mediterranean fever, eosinophilic sinusitis, dilated cardiomyopathy, food allergy, systemic mastocytosis, amyotrophic lateral sclerosis, inclusion body myositis and the like.

At least a part of the PD-1/CD4 bispecific protein (preferably a PD-1/CD4 bispecific antibody etc.) which can be used in treatment of autoimmune diseases specifically bind to PD-1 and CD4 expressed on lymphocytes (preferably T-cell lymphocytes (e.g., helper T cells and regulatory T cells)), respectively, and exert therapeutic effects by transmitting the PD-1 inhibitory signal. In addition, the feature of allowing the interaction with PD-1 and PD-L1 are expected to contribute to the enhancement or persistence of the effects of prevention, suppression of the progression of symptoms, or the recurrence and/or treatment.

Examples of hematological cancer in the present invention include multiple myeloma, malignant lymphoma (e.g., non-Hodgkin's Lymphoma (e.g., B-cell non-Hodgkin's lymphoma (e.g., precursor B-cell lymphoblastic lymphoma, precursor B-cell acute lymphoblastic leukemia, chronic B-lymphoid leukemia (small lymphocytic lymphoma or prolymphocytic leukemia), B-cell prolymphocytic leukemia, lymphoplasmacytic lymphoma, nodal marginal zone B-cell lymphoma, extranodal marginal zone B-cell lymphoma (MALT lymphoma), primary splenic marginal zone B-cell lymphoma, hairy cell leukemia, hairly cell leukemia-variant, follicular lymphoma, pediatric type follicular lymphoma, diffuse large B cell lymphoma, diffuse large B-cell lymphoma, not otherwise specified, splenic diffuse red pulp small B-cell lymphoma, lymphoplasmacytic lymphoma, primary mediastinal large B-cell lymphoma, primary effusion lymphoma, Burkitt's lymphoma, mantle cell lymphoma, monoclonal B-cell lymphocytosis, splenic B-cell lymphoma/leukemia, unclassifiable, IgM monoclonal gammopathy of undetermined significance, µ heavy chain disease, λ heavy chain disease, α heavy chain disease, plasma cell myeloma, solitary plasmacytoma of bone, extraosseous plasmacytoma, monoclonal immunoglobulin deposition disease, large B-cell lymphoma with IRF4 rearrangement, primary cutaneous follicle center lymphoma, T-cell/histiocyte-rich large B-cell lymphoma, primary diffuse large B-cell lymphoma of the central nervous system, primary cutaneous diffuse large B-cell lymphoma, leg type, EBV positive diffuse large B-cell lymphoma, not otherwise specified, EBV positive mucocutaneous ulcer, diffuse large B-cell lymphoma associated with chronic inflammation, lymphomatoid granulomatosis, intravascular large B-cell lymphoma, ALK positive large B-cell lymphoma, plasmablastic lymphoma, primary effusion lymphoma, HHV8 positive diffuse large B-cell lymphoma, not otherwise specified, Burkitt-like lymphoma with 11q aberration, high-grade B-cell lymphoma, with MYC and BCL2 and/or BCL6 rearrangement, high-grade B-cell lymphoma, not otherwise specified and B-cell lymphoma, unclassifiable, with intermediate features between diffuse large B-cell lymphoma and classical Hodgkin lymphoma), T/NK-cell non-Hodgkin's lymphoma (e.g., precursor T-cell lymphoblastic lymphoma, chronic T-cell lymphocytic leukemia, T-cell acute lymphocytic leukemia (T-cell lymphoblastic leukemia), T-cell large granular lymphoblastic lymphoma, NK-cell large granular leukemia, aggressive NK-cell leukemia, peripheral T-cell lymphoma, peripheral T-cell lymphoma, not otherwise specified, unclassifiable peripheral T-cell lymphoma, angioimmunoblastic T-cell lymphoma, (CD30-positive) anaplastic large cell lymphoma, angiocentric lymphoma, intestinal T-cell lymphoma, enteropathy-type T-cell lymphoma, hepatosplenic gamma-delta T cell lymphoma, subcutaneous panniculitis-like T-cell lymphoma, mycosis fungoides, Sezary syndrome, Hodgkin-like/Hodgkin-related anaplastic large cell lymphoma, extranodal NK/T-cell lymphoma, adult T cell leukemia, T-cell prolymphocytic leukemia, chronic lymphoproliferative disorder of NK-cells, systemic EBV positive T-cell lymphoma of childhood, hydroa vacciniforme-like lymphoproliferative disorder, extranodal NK/T-cell lymphoma, nasal type, enteropathy-associated T-cell lymphoma, monomorphic epitheliotropic intestinal T-cell lymphoma, indolent T-cell lymphoproliferative disorder of the GI tract, hepatosplenic T-cell lymphoma, primary cutaneous CD30 positive T-cell lymphoproliferative disorders, lymphomatoid papulosis, primary cutaneous anaplastic large cell lymphoma, primary cutaneous gamma-delta T-cell lymphoma, primary cutaneous CD8 positive aggressive epidermotropic cytotoxic T-cell lymphoma, primary cutaneous acral CD8 positive T-cell lymphoma, primary cutaneous CD4 positive small/medium T-cell lymphoproliferative disorder, follicular T-cell lymphoma, nodal peripheral T-cell lymphoma with TFH phenotype, anaplastic large cell lymphoma, ALK positive, anaplastic large cell lymphoma, ALK negative and breast implant-associated anaplastic large-cell lymphoma)), and Hodgkin's Lymphoma (e.g., classical Hodgkin's lymphoma (e.g., nodular sclerosis, mixed cellularity, lymphocyte-rich and lymphopenic) or nodular lymphocyte predominant Hodgkin's lymphoma)), leukemia (e.g., acute myelogenous leukemia, acute promyelocytic leukemia, acute lymphoblastic leukemia (lymphoblastic lymphoma), chronic lymphocytic leukemia myelodysplastic syndrome, and chronic myelogenous leukemia), primary central nervous system lymphoma, myeloproliferative syndrome and the like.

At least a part of the PD-1/CD4 bispecific protein (preferably a PD-1/CD4 bispecific antibody etc.) which can be used in the present invention for treatment of hematological cancer and the like specifically binds to PD-1 expressed on cancer cells of hematological cancer, which are target cells, and CD4 expressed on lymphocytes, which are effector cells (preferably T cell lymphocytes (e.g. helper T cells or regulatory T cells etc.) etc.), respectively, and exerts therapeutic effects thereof. Furthermore, at least a part thereof specifically binds to PD-1 and CD4 expressed on T-cell-derived cancer cells, respectively, and may exert therapeutic effects thereof by transmitting the PD-1 inhibitory signal.

In the present invention, the term "treating" means curing or improving certain disease or symptoms thereof. The term "preventing" means preventing the onset of diseases or symptoms thereof or delaying that for a certain period of time. The term "suppressing of the progression of symptoms" means suppressing the progress or aggravation of symptoms to stop the progress of disease conditions. The meaning of "preventing" also includes suppressing the recurrence. The term "suppressing the recurrence" means preventing the recurrence of certain disease or symptoms thereof or reducing a possibility of recurrence.

The PD-1/CD4 bispecific protein of the present invention is usually administered systemically or locally through parenteral administration. Specific examples of such administration methods include injection administration, intranasal administration, transpulmonary administration and the like. Examples of injection administration include intravenous injection, intramuscular injection, intraperitoneal injection and the like. For intravenous injection, drip intravenous infusion is preferable. The dosage thereof varies depending on the age, body weight, symptoms, therapeutic effect, administration method, treating period and the like, but it is usually for an adult patient within a range of 0.1 µg/kg to 300 mg/kg per dose, particularly preferably within a range of 0.1 mg/kg to 10 mg/kg, once to several times per day by parenteral administration, or within a range of 30 minutes to 24 hours per day by intravenous sustaining administration. Needless to say, as mentioned above, since the dose varies depending on various conditions, it may be lower than the above-mentioned dose, or may be needed to be more than the above.

### Formulation

If the PD-1/CD4 bispecific protein of the present invention is formulated to be used as an injection or infusion solution for drip infusion, the injection or infusion solution may be in any form of an aqueous solution, suspension or emulsion, or may be formulated as a solid agent along with pharmaceutically acceptable carriers such that it can be dissolved, suspended or emulsified by adding a solvent at the time of use. Examples of solvents which can be used in the injection or the infusion solution for drip infusion include distilled water for injection, physiological saline, glucose solutions and isotonic solutions and the like (e.g., solutions in which sodium chloride, potassium chloride, glycerin, mannitol, sorbitol, boric acid, borax, propylene glycol or the like is dissolved.).

Herein, examples of the pharmaceutically acceptable carriers include a stabilizer, solubilizer, suspending agent, emulsifier, soothing agent, buffering agent, preservative, antiseptic agent, pH adjuster, antioxidant and the like. As a stabilizer, for example, various amino acids, albumin, globulin, gelatin, mannitol, glucose, dextran, ethylene glycol, propylene glycol, polyethylene glycol, ascorbic acid, sodium bisulfite, sodium thiosulfate, sodium edetate, sodium citrate, dibutylhydroxytoluene or the like can be used. As a solubilizer, for example, alcohol (e.g., ethanol etc.), polyalcohol (e.g., propylene glycol and polyethylene glycol, etc.), nonionic surfactant (e.g., Polysorbate 20 (registered trademark), Polysorbate 80 (registered trademark) and HCO-50, etc.) or the like can be used. As a suspending agent, for example, glyceryl monostearate, aluminum monostearate, methyl cellulose, carboxymethyl cellulose, hydroxymethyl cellulose, sodium lauryl sulfate or the like can be used. As an emulsifier, for example, gum arabic, sodium alginate, tragacanth or the like can be used. As a soothing agent, for example, benzyl alcohol, chlorobutanol, sorbitol or the like can be used. As a buffering agent, for example, phosphate buffer, acetate buffer, borate buffer, carbonate buffer, citrate buffer, Tris buffer, glutamic acid buffer, epsilon aminocaproic acid buffer or the like can be used. As a preservative, for example, methyl parahydroxybenzoate, ethyl parahydroxybenzoate, propyl parahydroxybenzoate, butyl parahydroxybenzoate, chlorobutanol, benzyl alcohol, benzalkonium chloride, sodium dehydroacetate, sodium edeate, boric acid, borax or the like can be used. As an antiseptic agent, for example, benzalkonium chloride, parahydroxybenzoic acid, chlorobutanol or the like can be used. As a pH adjuster, for example, hydrochloric acid, sodium hydroxide, phosphoric acid, acetic acid or the like can be used. As an antioxidant, for example, (1) aqueous antioxidants such as ascorbic acid, cysteine hydrochloride, sodium bisulfate, sodium metabisulfite and sodium sulfite, (2) oil-soluble antioxidants such as ascorbyl palmitate, butylated hydroxy anisole, butylated hydroxyl toluene, lecithin, propyl gallate and α-tocopherol and (3) metal chelating agents such as citric acid, ethylenediaminetetraacetic acid, sorbitol, tartaric acid and phosphoric acid can be used.

The injection or infusion solution for drip infusion can be prepared by performing sterilization in the final process, or aseptic manipulation, for example, sterilization by filtration with a filter or the like and subsequently filling it to an aseptic container. The injection or infusion solution for drip infusion may be used by dissolving the vacuum dried and lyophilized aseptic powder (which may include pharmaceutically acceptable carrier powders) in an appropriate solvent at the time of use.

### Combination use or combination formulation

The PD-1/CD4 bispecific protein of the present invention may be used in combination with other agents which is used for preventing, suppressing the progression of symptoms of or the recurrence of and/or treating autoimmune disease, graft-versus-host disease (GVHD) or hematological cancer. In the present invention, examples of administration forms in combinational use with other agents (combinational use) may include a form of combination formulation containing both of ingredients in one formulation and a form being administered in separate formulations. Such combinational uses can complement the effect on preventing, suppressing the progression of symptoms of, suppressing the recurrence of and/or treating by other agents, or can maintain or reduce the dose or frequency of administration of other agents. If separately administering the bispecific antibody of the present invention and other agents, they may be simultaneously administered for a certain period of time, and then only the bispecific antibody or other agents may be administered. Alternatively, the bispecific antibody of the present invention may be initially administered, and after completion of administration thereof, other agents may be administered. Other agents may be initially administered, and after completion of administration thereof, the bispecific antibody of the present invention may be administered. The respective administration methods thereof may be the same as or different from each other. A kit containing a formulation containing the bispecific antibody of the present invention and a formulation containing other agents can also be provided. Herein, the dosage of other agents can be appropriately selected based on the dosage used in clinical use. Further, other agents may be administered in combination of two or more kinds of arbitrary agents at an appropriate ratio. Furthermore, examples of other agents include not only those already known but also those newly discovered in the future.

For example, if applying the bispecific protein of the present invention to prevent, suppress the progression of symptoms of or the recurrence of and/or treat type I diabetes mellitus, it may be used in combination with an insulin preparation (e.g., human insulin, insulin glargine, insulin lispro, insulin detemir, insulin aspart, etc.), sulfonylurea agent (e.g., Glibenclamide, Gliclazide and Glimepiride, etc.), quick-acting insulin secretion promoter (e.g., Nateglinide etc.), biguanide preparation (e.g., Metformin etc.), insulin sensitizer (e.g., Pioglitazone etc.), α-glucosidase inhibitor (e.g., Acarbose and Voglibose, etc.), diabetic neuropathy therapeutic agent (e.g., Epalrestat, Mexiletine and Imidapril, etc.), GLP-1 analog preparation (e.g., Liraglutide, Exenatide and Lixisenatide, etc.) or DPP-4 inhibitor (e.g., Sitagliptin, Vildagliptin and Alogliptin, etc.).

Furthermore, for example, if applying the bispecific protein of the present invention to prevent, suppress the progression of symptoms of or the recurrence of and/or treat multiple sclerosis, it may be used in combination with a steroid drug (e.g., cortisone, cortisone acetate, hydrocortisone, hydrocortisone sodium phosphate, hydrocortisone sodium succinate, fludrocortisone acetate, prednisolone, prednisolone acetate, prednisolone sodium succinate, prednisolone butylacetate, prednisolone sodium phosphate, halopredone acetate, methylprednisolone, methylprednisolone acetate, methylprednisolone sodium succinate, triamcinolone, triamcinolone acetate, triamcinolone acetonide, dexamethasone, dexamethasone acetate, dexamethasone valerate, dexamethasone cipecilate, dexamethasone palmitate, dexamethasone propionate, dexamethasone sodium phosphate, dexamethasone sodium metasulfobenzoate, paramethasone, paramethasone acetate, betamethasone, betamethasone dipropionate, betamethasone valerate, betamethasone acetate, betamethasone butyrate propionate and betamethasone sodium phosphate, etc.), interferon β-1a, interferon β-1b, glatiramer acetate, Mitoxantrone, Azathioprine, Cyclophosphamide, Ciclospolin, Methotrexate, Cladribine, adrenocorticotropic hormone (ACTH), Corticotropin, Mizoribine, Tacrolimus, Fingolimod, Alemtuzumab and the like.

In addition, for example, if applying the bispecific protein of the present invention to prevent, suppress the progression of symptoms of or the recurrence of and/or treat systemic lupus erythematosus, it may be used in combination with a steroid drug (e.g., steroid drugs mentioned above) or immunosuppressive agent (e.g., Ciclospolin, Tacrolimus, Fingolimod and the like).

For example, if applying the bispecific protein of the present invention to prevent, suppress the progression of symptoms of or the recurrence of and/or treat rheumatoid arthritis, it may be used in combination with a steroid drug (e.g., steroid drugs mentioned above), anti-rheumatic drug (e.g., Methotrexate, Sulfasalazine, Bucillamine, Leflunomide, Mizoribine, Tacrolimus or the like), anti-cytokine drug (e.g., Infliximab, Adalimumab, Tocilizumab, Etanercept, Abatacept, Golimumab Certolizumab or the like) or the like.

If applying to prevent, suppress the progression of symptoms of or the recurrence of and/or treat other autoimmune diseases, the bispecific antibody of the present invention may be used in combination with any one or more of the above-mentioned other drugs.

On the other hand, if applying the bispecific protein of the present invention to prevent, suppress the progression of symptoms of or the recurrence of and/or treat hematological cancer, it may be used in combination with, for example, any one or more of agents selected from an alkylating agent (e.g., Dacarbazine, Nimustine, Temozolomide, Fotemustine, Bendamustine, Cyclophosphamide, Ifosfamide, Carmustine, Chlorambucil and Procarbazine, etc.), platinum agent (e.g., Cisplatin, Carboplatin, Nedaplatin and Oxaliplatin, etc.), antimetabolite (e.g., antifolate (e.g., Pemetrexed, Leucovorin and Methotrexate, etc.), pyridine metabolism inhibitor (e.g., TS-1 (registered trademark), 5-fluorouracil, UFT, Carmofur, Doxifluridine, FdUrd, Cytarabine and Capecitabine, etc.), purine metabolism inhibitor (e.g., Fludarabine, Cladribine and Nelarabine, etc.), ribonucleotide reductase inhibitor, nucleotide analog (e.g., Gemcitabine etc.)), topoisomerase inhibitor (e.g., Irinotecan, Nogitecan and Etoposide, etc.), microtubule polymerization inhibitor (e.g., Vinblastine, Vincristine, Vindesine, Vinorelbine and Eribulin, etc.), microtubule depolymerization inhibitor (e.g., Docetaxel and Paclitaxel, etc.), antitumor antibiotic (e.g., Bleomycin, Mitomycin C, Doxorubicin, Daunorubicin, Idarubicin, Etoposide, Mitoxantrone, Vinblastine, Vincristine, Peplomycin, Amrubicin, Aclarubicin and Epirubicin, etc.), cytokine preparation (e.g., IFN-α2a, IFN-α2b, pegylated IFN-α2b, natural IFN-β and interleukin-2, etc.), molecular targeting drug, cancer immunotherapeutic drug, other antibody drugs and the like.

Herein, examples of the molecular targeting drugs include an ALK inhibitor (e.g., Crizotinib, Ceritinib, Ensartinib, Alectinib and Lorlanib, etc.), BCR-ABL inhibitor (e.g., Imatinib and Dasatinib, etc.), AXL inhibitor (e.g., ONO-7475 and BGB324, etc.), CDK inhibitor (e.g., Dinaciclib, Abemaciclib, Palbociclib and Trilaciclib, etc.), BTK inhibitor (e.g., Ibrutinib and Acarabultinib, etc.), PI3K-δ/γ inhibitor (e.g., Umbralisib, Parsaclisib and IPI-549, etc.), JAK-1/2 inhibitior (e.g., Itacitinib and Ruxolitinib, etc.), FAK inhibitor (e.g., Defactinib etc.), Syk/FLT3 dual inhibitor (e.g., Mivavotinib etc.), ATR inhibitor (e.g., Ceralasertib etc.), Wee1 kinase inhibitor (e.g., Adavosertib etc.), mTOR inhibitor (e.g., Temsirolimus, Everolimus, Vistusertib and Irinotecan, etc.), HDAC inhibitor (e.g., Vorinostat, Romidepsin, Entinostat, Chidamide, Mocetinostat, Citarinostat, Panobinostat and Valproate, etc.), EZH2 Inhibitor (e.g., Tazemetostat etc.), STAT3 Inhibitor (e.g., Napabucasin etc.), DNMT Inhibitor (e.g., Azacitidine etc.), BCL-2 inhibitor (e.g., Navitoclax and Venetoclax, etc.), SMO Inhibitor (e.g., Vismodegib etc.), anti-CD20 antibody (e.g., Rituximab, Blontuvetmab, Epitumomab, Ibritumomab tiuxetan, Ocaratuzumab, Ocrelizumab, Technetium (⁹⁹mTc) nofetumomab merpentan, Tositumomab, Veltuzumab, Ofatumumab, Ublituximab, Obinutuzumab and Nofetumomab, etc.), anti-CD30 antibody (e.g., Brentuximab vedotin and Iratumumab, etc.), anti-CD38 antibody (e.g., Daratumumab, Isatuximab, Mezagitamab, AT13/5 and MOR202, etc.), anti-DR5 antibody (e.g., DS-8273a etc.), anti-TNFRSFlOB antibody (e.g., Benufutamab, Conatumumab, Drozitumab, Lexatumumab, Tigatuzumab, Eftozanermin alfa and DS-8273a, etc.), anti-CD40 antibody (e.g., Bleselumab, Dacetuzumab, Iscalimab, Lucatumumab, Mitazalimab, Ravagalimab, Selicrelumab, Teneliximab, ABBV-428 and APX005M, etc.), anti-CD70 antibody (e.g., Cusatuzumab, Vorsetuzumab, Vorsetuzumab mafodotin and ARGX-110, etc.), anti-TNFRSF10A antibody (e.g., Mapatumumab etc.), anti-CD79b antibody (e.g., Iladatuzumab, Iladatuzumab vedotin and Polatuzumab vedotin, etc.), anti-TNFSF11 antibody (e.g., Denosumab etc.), anti-NCAM1 antibody (e.g., Lorvotuzumab mertansine etc.), anti-CD37 antibody (e.g., Lilotomab, Lutetium (¹⁷⁷lu) lilotomab satetraxetan, Naratuximab, Naratuximab emtansine and Otlertuzumab, etc.), anti-CD200 antibody (e.g., Samalizumab etc.), anti-CD30-CD16A bispecific antibody (e.g., AFM13 etc.), anti-IL3RA-CD3 bispecific antibody (Flotetuzumab and Vibecotamab), anti-GPRC5D-CD3 bispecific antibody (e.g., Talquetamab), anti-CD3-CD19 bispecific antibody (e.g., Duvortuxizumab and Blinatumomab, etc.), anti-TNFRSF17-CD3 bispecific antibody (e.g., Teclistamab), anti-CLEC12A-CD3 bispecific antibody (e.g., Tepoditamab) and anti-CD20-CD3 bispecific antibody (e.g., Plamotamab, Odronextamab, Mosunetuzumab, Glofitamab, Epcoritamab and REGN1979, etc.) and the like.

Furthermore, examples of the cancer immunotherapeutic drugs include an anti-PD-1 antibody (e.g., Nivolumab, Cemiplimab, Penbrolizumab, Spartalizumab, Tislelizumab, Dostarlimab, Toripalimab, Camrelizumab, Genolimzumab, Sintilimab, Lodapolimab, Retifanlimab, Balstilimab, Serplulimab, Budigalimab, Prolgolimab, Sasanlimab, Cetrelimab, Zimberelimab, Penpulimab, AMP-514, STI-A1110, ENUM 388D4, ENUM 244C8, GLS1010, CS1003, BAT-1306, AK103, BI 754091, LZM009, CMAB819, Sym021, SSI-361, JY034, ISU106, HX008 and CX-188, etc.), anti-PD-L1 antibody (e.g., Atezolizumab, Avelumab, Durvalumab, Manelimab, Pacmilimab, Envafolimab, Cosibelimab, BMS-936559, STI-1010, STI-1011, STI-1014, SHR-1316, CS1001, MSB2311, BGB-A333, KL-A167, AK106, AK104, ZKAB001, FAZ053, CBT-502 and JS003, etc.), PD-1 antagonist (e.g., AUNP-12, the respective compounds of BMS-M1 to BMS-M10, BMS-1, BMS-2, BMS-3, BMS-8, BMS-37, BMS-200, BMS-202, BMS-230, BMS-242, BMS-1001, BMS-1166, the respective compounds of Incyte-1 to Incyte-6, the respective compounds of CAMC-1 to CAMC-4, RG_1 and DPPA-1, etc.), PD-L1/VISTA antagonist (e.g., CA-170 etc.), PD-L1/TIM3 antagonist (e.g., CA-327 etc.), anti-PD-L2 antibody, PD-L1 fusion protein, PD-L2 fusion protein (e.g., AMP-224 etc.), anti-CTLA-4 antibody (e.g., Ipilimumab, Zalifrelimab, Nurulimab and Tremelimumab, etc.), anti-LAG-3 antibody (e.g., Relatlimab, Ieramilimab, Fianlimab, Encelimab and Mavezelimab, etc.), anti-TIM3 antibody (e.g., MBG453 and Cobolimab, etc.), anti-KIR antibody (e.g., Lirilumab, IPH2101, LY3321367 and MK-4280, etc.), anti-BTLA antibody, anti-TIGIT antibody (e.g., Tiragolumab, Etigilimab, Vibostolimab and BMS-986207, etc.), anti-VISTA antibody (e.g., Onvatilimab etc.), anti-CD137 antibody (e.g., Urelumab and Utomilumab, etc.), anti-CSF-1R antibody or CSF-1R inhibitor (e.g., Cabiralizumab, Ecactuzumab, LY3022855, Axatilimab, MCS-110, IMC-CS4, AMG820, Pexidartinib, BLZ945 and ARRY-382, etc.), anti-OX40 antibody (e.g., MEDI 6469, Ivuxolimab, MEDI0562, MEDI6383, Efizonerimod, GSK3174998, BMS-986178 and MOXR0916, etc.), anti-HVEM antibody, anti-CD27 antibody (e.g., Varlilumab etc.), anti-GITR antibodies or GITR fused protein (e.g., Efaprinermin alfa, Efgivanermin alfa, MK-4166, INCAGN01876, GWN323 and TRX-518, etc.), anti-CD28 antibody, anti-CCR4 antibody (e.g., Mogamulizumab etc.), anti-B7-H3 antibody (e.g., Enoblituzumab, Mirzotamab, Mirzotamab clezutoclax and Omburtamab, etc.), anti-ICOS agonist antibody (e.g., Vopratelimab and GSK3359609, etc.), anti-CD4 antibody (e.g., Zanolimumab and IT1208, etc.), anti-DEC-205 antibody/NY-ESO-1 fusion protein (e.g., CDX-1401 etc.), anti-SLAMF7 antibody (e.g., Azintuxizumab, Azintuxizumab vedotin and Elotuzumab, etc.), anti-CD73 antibody (e.g., Oleclumab and BMS-986179, etc.), pegylated IL-2 (e.g., Bempegaldesleukin), anti-CD40 agonist antibody (e.g., ABBV-428, APX005M and RO7009789, etc.), IDO inhibitor (e.g., Epacadostat, Indoximod and Linrodostat, etc.), TLR agonist (e.g., Motolimod, CMP-001, G100, Tilsotolimod, SD-101 and MEDI9197, etc.), adenosine A2A receptor antagonist (e.g., Preladenant, AZD 635, Taminadenant and Ciforadenant, etc.), anti-NKG2A antibody (e.g., Monalizumab etc.), anti-CSF-1 antibody (e.g., PD0360324 etc.), immunopotentiating agent (e.g., PV-10 etc.), IL-15 superagonist (e.g., ALT-803), soluble LAG3 (e.g., IMP 321 etc.), anti-CD47 antibody or CD47 antagonist (e.g., ALX148 etc.), IL-12 antagonist (e.g., M9241 etc.) and the like.

Furthermore, examples of the other antibody drugs include anti-IL-1β antibodies (e.g., Canakinumab etc.), anti-CCR2 antibodies (e.g., Plozalizumab etc.) and the like.

The present invention will now be described in more detail by the following examples, but the scope of the present invention is not limited thereto. A person skilled in the art can make various changes and modifications, based on the description of the present invention, and such changes and modifications are also included in the present invention.

### [Examples]

### Example 1: Preparation of the PD-1/CD4 bispecific protein (PD-1/CD4 scDb)

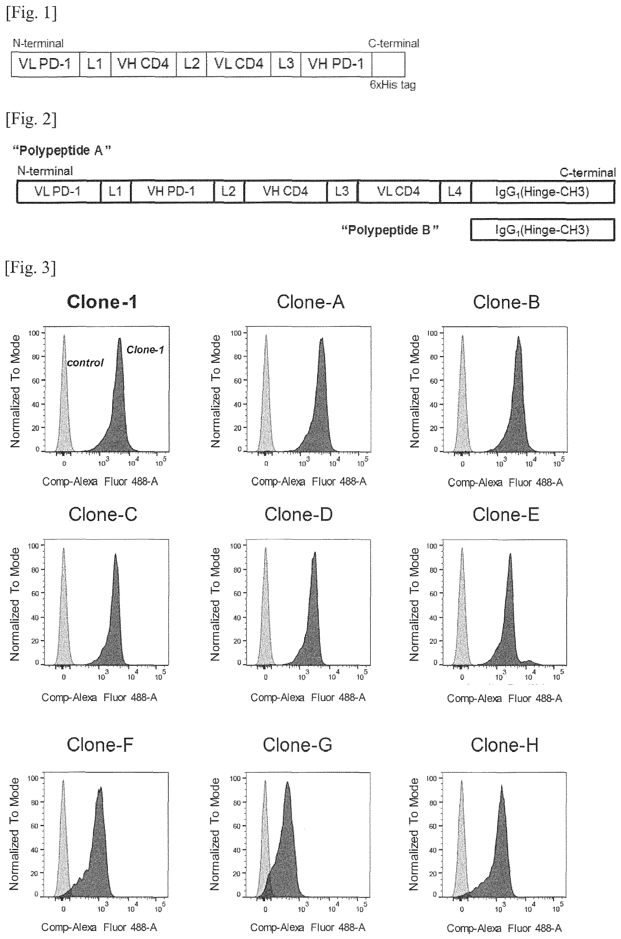

The PD-1/CD4 bispecific protein PD-1/CD4 scDb in the present example is a single-chain Diabody composed of the VH and VL of anti-mouse CD4 antibody and the VH and VL of anti-mouse PD-1 antibody (hereinafter, be abbreviated as "scDb"). Figure 1 shows a schematic of structure thereof. The scDb was expressed in animal cells (Free Style 293F cells) by inserting the DNA generated by linking DNAs encoding the variable regions of each antibody and peptide linkers in a predetermined order into an expression vector. The scDb produced in culture supernatant was collected and purified by Ni-NTA affinity chromatography and size exclusion chromatography. There were nine PD-1/CD4 scDb prepared in the present example, and one of them was designated as Clone-1. The cDNA encoding each variable region (VH and VL) of anti-mouse CD4 antibody and anti-mouse PD-1 antibody, composed of the scDb, respectively, in the present example was obtained according to methods based on known gene cloning methods. In the present example, as an example, J43 was used as an anti-mouse PD-1 antibody and YTA3.1.2, YTS191 or GK1.5 as an anti-mouse CD4 antibody.

### Example 2: Preparation of the PD-1 × CD4 bispecific protein (PD-1/CD4 scDb-Fc)

The PD-1/CD4 scDb-Fc in the present example is a heterodimer composed by the association of two proteins which are a polypeptide (hereinafter, be abbreviated as Polypeptide A) composed by fusing the human IgG₁ constant region from the hinge site to the CH3 region (IgGi constant region (hinge/CH3)) to a bispecific scDb consisting of the VH and VL of anti-mouse CD4 antibody and the VH and VL of anti-mouse PD-1 antibody, and a polypeptide consisting of only the IgG₁ constant region (hinge/CH3) (hereafter be abbreviated as Polypeptide B). The heterodimer has a mutation eliminating the binding property to Fcγ receptor. Figure 2 shows a schematic of structure thereof.

In producing PD-1/CD4 scDb, a DNA encoding Polypeptide A was prepared by linking DNAs encoding the VH and VL of anti-mouse CD4 antibody and anti-mouse PD-1 antibody obtained in Example 1, respectively, with DNAs encoding the peptide linker in a predetermined order. A cDNA encoding the IgG1 constant region (hinge/CH3), constituting a part of Polypeptide A and B, was obtained by PCR based on known genetic information. Expression vectors containing DNAs encoding Polypeptides A and B were genetically transferred into Free Style 293F cells and expressed to produce in culture supernatant. It was prepared by collecting the culture supernatant and then purifying by Protein A affinity chromatography and size exclusion chromatography. There are nine types of PD-1/CD4 scDb-Fc prepared in the present example, and one of them is designated as "Clone-2".

### Example 3: Binding evaluation of the PD-1/CD4 bispecific protein

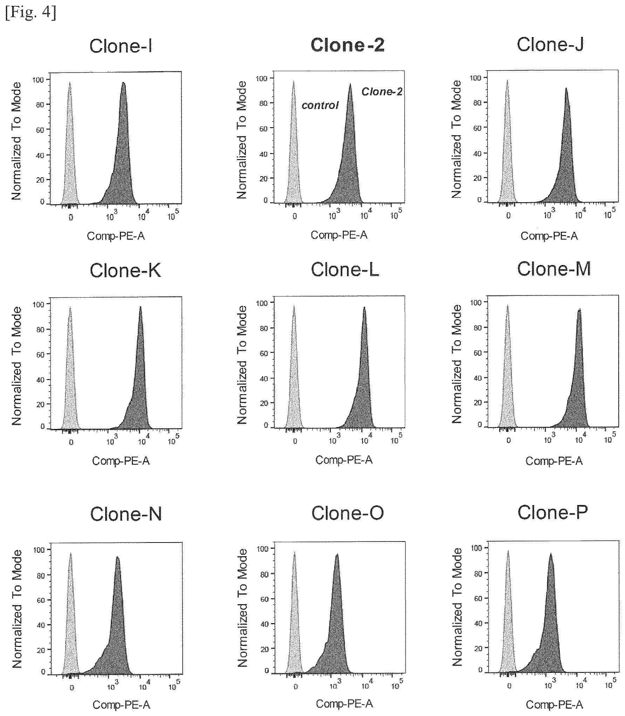

The binding of PD-1/CD4 bispecific protein to mouse PD-1 was evaluated by flow cytometry using mouse PD-1 expressing CHO-S cells. A 6 × His tag was added to the C-terminus of PD-1/CD4 scDb, and Alexa Fluor 488-conjugated anti-His tag antibody (MBL, model number D291-A48) was used to detect each recombinant protein bound to the same cells. Figure 3 shows results thereof. On the other hand, since PD-1/CD4 scDb-Fc contains the human IgG₁ constant region, PE-conjugated anti-human IgG-Fc antibody (Thermo Fisher Scientific, model number H10104) was used to detect the binding to mouse PD-1. Figure 4 shows results thereof.

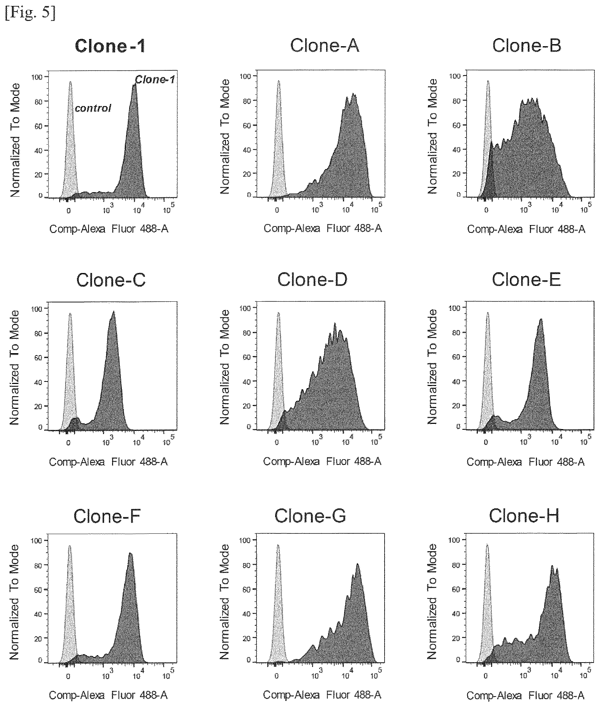

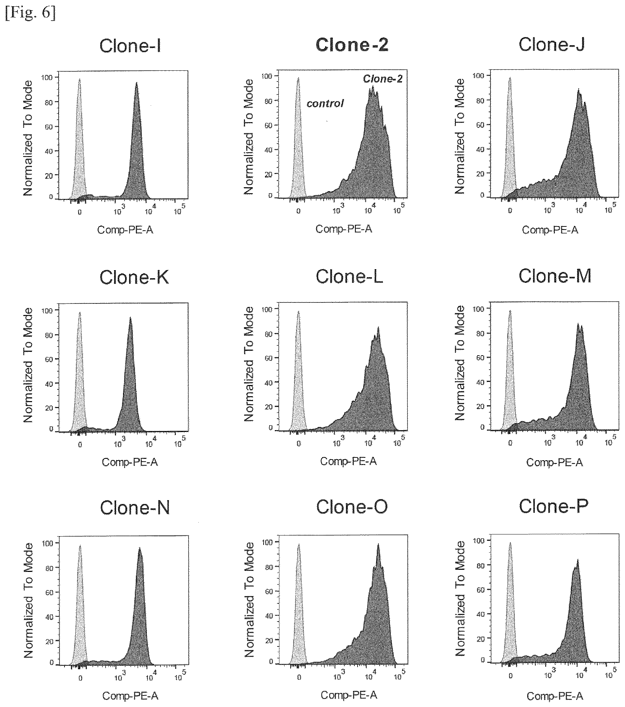

It was confirmed that all of the PD-1/CD4 bispecific proteins also binds to mouse PD-1 expressing CHO-S cells. On the other hand, no binding to CHO-S cells used as control, was observed, which confirmed that the binding was specific to expressed PD-1. Then, it was confirmed, by the same method, that the PD-1/CD4 bispecific protein specifically binds to mouse CD4 using mouse CD4-expressing CHO-K1 cells. Figures 5 and 6 show results thereof.

### Example 4: In vivo effects of the PD-1/CD4 bispecific protein in the experimental allergic encephalomyelitis mouse model (EAE model)

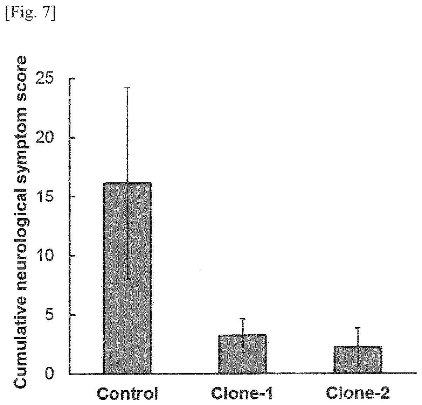

In vivo effects of the PD-1/CD4 bispecific protein were evaluated in the EAE model using C57BL/6 mice. Killed mycobacterium tuberculosis H37Ra (BD Biosciences, serial number 231141) and incomplete Freund's adjuvant (BD Biosciences, serial number 263910) were mixed with each other to prepare a complete Freund's adjuvant (CFA) containing 4 mg/mL killed mycobacterium tuberculosis H37Ra. 1 mg/mL MOG peptide (ANASPEC, serial number AS-60130) and the equal amount of CFA were mixed with each other to prepare emulsion as an eliciting agent of the EAE model. 200 µL of the eliciting agent was subcutaneously administered to tail head of the same C57BL/6 mice. On the day of immunization and on day 2,200 µL of 1 µg/mL of pertussis toxin (SIGMA-ALDRICH, serial number P7208) were administered in tail vein, respectively. Next, on 5 days from day 6 to 10 since immunization, Clone-1 or Clone-2 was intraperitoneally administered to the same C57BL/6 mice once a day. The neurological symptoms after immunization were evaluated in accordance with the method of Onuki, et al. (Onuki M,et al., Microsc Res Tech 2001; 52: 731-9). The degrees of neurological symptoms were scored (normal: score 0, tail relaxation: score 1, hind limb partial paralysis: score 2, hind limb paralysis: score 3, forelimb paralysis: score 4 and dying or death: score 5). If a plurality of neurological symptoms were observed, the higher score was employed as the neurological symptom on the evaluation day. The cumulative neurological symptom scores for the observation period from the day of immunization to 30 days post-immunization were recorded. Figure 7 shows results thereof.

Clone-1 and Clone-2 suppressed the neurological symptoms in the EAE model.

### Example 5: Effect of PD-1/CD4 bispecific protein on in vivo cytokine levels in mouse blood

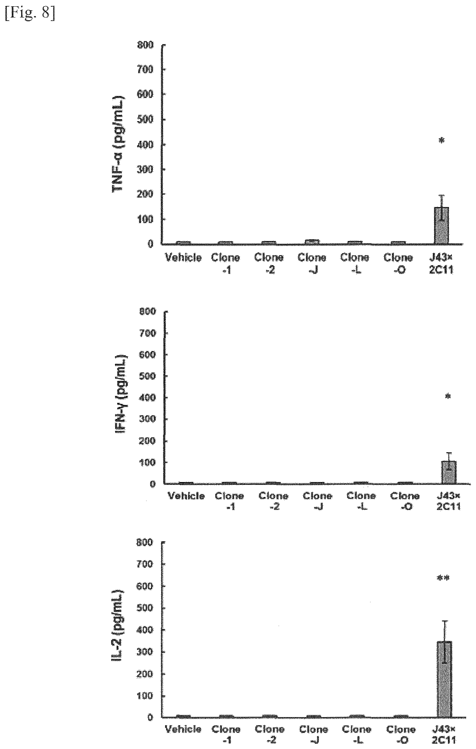

The change in plasma cytokine levels *in vivo* was evaluated by administering the PD-1/CD4 bispecific protein of the present invention to mice. 0.5 mg/kg of Clone-1 and 1.0 mg/kg of Clone-2, Clone-J, Clone-L, and Clone-O as the PD-1/CD4 bispecific protein were administered to C57BL/6 mice, respectively. In addition, 0.5 mg/kg of the PD-1/CD3 bispecific scDb (J43×2C11_scDb) disclosed in Patent Literature 1 was administered intraperitoneally. Three hours after each administration, blood was drawn from the tail vein of mice and plasma was separated. Plasma concentrations of interferon-γ (IFN-γ), tumor necrosis factor-α (TNF-α), and interleukin-2 (IL-2) were measured with flow cytometer (BD Biosciences, BD FACSCantoII) using mouse soluble protein Flex Set of BD Cytometric Bead Array (BD Biosciences, model numbers 558296, 558299 and 558297). Figure 8 shows results thereof. Plasma IFN-γ, TNF-α, and IL-2 concentrations (pg/mL) in the figure are shown as mean ± standard error (N=5).

J43×2C11_scDb increased plasma IFN-γ, TNF-α, and IL-2 levels in mice. On the other hand, none of the PD-1/CD4 bispecific protein of the present invention increased plasma IFN-γ, TNF-α, and IL-2 levels. When the target molecule was CD4, the induction of cytokine production or release upon administration was improved.

### Example 6: Suppressive effects of the PD-1/CD4 bispecific protein against cell proliferation of B cell lymphoma cell lines

Using pan-T cells (STEMCELL, serial number ST-70024) (human T cells) derived from peripheral blood of healthy persons, the suppressive effect against cell proliferation of B cell lymphoma cell lines is evaluated. On U-bottom 96-well plates, human T cells and B-cell lymphoma cell line EB-1 or human PD-1-forced expressing SU-DHL-4 labeled with Vybrant DiD Cell-Labeling Solution (Thermo Fisher, serial number V22887) are seeded, and the PD-1/CD4 bispecific protein is added thereto, and then co-cultured for 48 hours. The cells are collected from each well, stained with Cell Viability Solution, and the number of DiD-positive viable cells is counted by flow cytometer. The number of DiD-positive living cells in the PD-1/CD4 bispecific protein-free sample is set to 100%, and the reduction rates of DiD-positive living cells in the samples to which the same protein is added are calculated as the suppression rate against cell proliferation.

### Example 7: Suppressive effects of the PD-1/CD4 bispecific protein against cell proliferation of multiple myeloma cell line

Using pan-T cells (STEMCELL, serial number ST-70024) (human T cells) derived from peripheral blood of healthy persons, the suppressive effect against cell proliferation of multiple myeloma cell line is evaluated. On U-bottom 96-well plates, human T cells and multiple myeloma cell line RPMI8226 labeled with Vybrant DiD Cell-Labeling Solution (Thermo Fisher, serial number V22887) are seeded, and the PD-1/CD4 bispecific recombinant protein is added thereto, and then co-cultured for 48 hours. The cells are collected from each well, stained with Cell Viability Solution, and the number of DiD-positive viable cells is counted by flow cytometer. The number of DiD-positive living cells in the PD-1/CD4 bispecific protein-free sample is set to 100%, and the reduction rate of DiD-positive living cells in the sample to which the same protein is added is calculated as the suppression rate against cell proliferation.

### Example 8: Suppressive effects of the PD-1/CD4 bispecific protein against cell proliferation of adult T-cell lymphoma cell line

Using pan-T cells (STEMCELL, serial number ST-70024) (human T cells) derived from peripheral blood of healthy persons, the suppressive effect on cell proliferation of adult T-cell lymphoma cell line is evaluated. On U-bottom 96-well plates, human T cells and adult T-cell lymphoma cell line ILT-Mat labeled with Vybrant DiD Cell-Labeling Solution (Thermo Fisher, serial number V22887) are seeded, and the PD-1/CD4 bispecific recombinant protein is added thereto, and then co-cultured for 48 hours. The cells are collected from each well, stained with Cell Viability Solution, and the number of DiD-positive viable cells is counted by flow cytometer. The number of DiD-positive living cells in the PD-1/CD4 bispecific protein-free sample is set to 100%, and the reduction rate of DiD-positive living cells in the sample to which the same protein is added is calculated as the suppression rate against cell proliferation.

### Example 9: Suppressive effects of the PD-1/CD4 bispecific protein against cell proliferation of acute T cell leukemia cell line

Using pan-T cells (STEMCELL, serial number ST-70024) (human T cells) derived from peripheral blood of healthy persons, the suppressive effect on cell proliferation of adult T-cell lymphoma cell line is evaluated. On U-bottom 96-well plates, human T cells and human PD-1-forced expressing Jurkat (acute T cell leukemia cell line) labeled with Vybrant DiD Cell-Labeling Solution (Thermo Fisher, serial number V22887) are seeded, and the PD-1/CD4 bispecific protein is added thereto, and then co-cultured for 48 hours. The cells are collected from each well, stained with Cell Viability Solution, and the number of DiD-positive viable cells is counted by flow cytometer. The number of DiD-positive living cells in the PD-1/CD4 bispecific protein-free sample is set to 100%, and the reduction rate of DiD-positive living cells in the sample to which the same protein is added is calculated as the suppression rate against cell proliferation.

### [Industrial Applicability]

The PD-1/CD4 bispecific protein of the present invention is useful for preventing, suppressing the progression of symptoms of or the recurrence of and/or treating autoimmune disease, graft-versus-host disease (GVHD) or hematological cancer.

## Claims

1. A PD-1/CD4 bispecific antibody or an antibody fragment thereof having a first arm specifically binding to PD-1 and a second arm specifically binding to CD4.

2. The PD-1/CD4 bispecific antibody or antibody fragment thereof according to claim 1, wherein the first arm comprises a VH and VL of anti-PD-1 antibody.

3. The PD-1/CD4 bispecific antibody or antibody fragment thereof according to claim 1 or 2, wherein the second arm comprises a VH and VL of anti-CD4 antibody.

4. The PD-1/CD4 bispecific antibody or antibody fragment thereof according to any one of claims 1to 3, which is an IgG antibody.

5. The PD-1/CD4 bispecific antibody or antibody fragment thereof according to claim 4, wherein the IgG antibody is an IgG₁ antibody or IgG₄ antibody.

6. The PD-1/CD4 bispecific antibody or antibody fragment thereof according to any one of claims 1 to 5, which transmits an inhibitory signal of PD-1.

7. The PD-1/CD4 bispecific antibody or antibody fragment thereof according to any one of claims 1 to 6, wherein the first arm allows an interaction with PD-1 and PD-L1, interaction with PD-1 and PD-L2, or both of interactions thereof.

8. The PD-1/CD4 bispecific antibody or antibody fragment thereof according to any one of claims 1 to 7, wherein cytokine production during or within 24 hours after administration is sufficiently reduced.

9. The PD-1/CD4 bispecific antibody or antibody fragment thereof according to any one of claims 1 to 8, which specifically binds to PD-1 and CD4 expressed on T lymphocytes, respectively.

10. An isolated PD-1/CD4 bispecific antibody having a first arm specifically binding to PD-1 and a second arm specifically binding to CD4,
(a) wherein the first arm comprises a VH and VL of anti-PD-1 antibody, and the second arm comprises a VH and VL of anti-CD4 antibody,
(b) which specifically binds to PD-1 and CD4 expressed on T lymphocytes, respectively, and
(c) (i) which transmits an inhibitory signal of PD-1 and (ii) wherein the first arm allows an interaction with PD-1 and PD-L1, interaction with PD-1 and PD-L2, or both of interactions thereof, and/or (iii) wherein cytokine production during or within 24 hours after administration is sufficiently reduced.

11. A pharmaceutical composition comprising the PD-1/CD4 bispecific antibody or antibody fragment thereof selected from any one of claims 1 to 10 and a pharmaceutically acceptable carrier.

12. An agent for preventing, suppressing the progression of symptoms of or the recurrence of and/or treating autoimmune disease or graft-versus-host disease, comprising the PD-1/CD4 bispecific antibody or antibody fragment thereof selected from any one of claims 1 to 10 as an active ingredient.

13. A method for preventing, suppressing the progression of symptoms of or the recurrence of and/or treating autoimmune disease or graft-versus-host disease, comprising administering an effective amount of the PD-1/CD4 bispecific antibody or antibody fragment thereof selected from any one of claims 1 to 10 to a patient.

14. A PD-1/CD4 bispecific antibody or antibody fragment thereof selected from any one of claims 1 to 10, for use in preventing, suppressing the progression of symptoms of or the recurrence of and/or treating autoimmune disease or graft-versus-host disease.

15. Use of a PD-1/CD4 bispecific antibody or antibody fragment thereof selected from any one of claims 1 to 10 for manufacturing a pharmaceutical agent for preventing, suppressing the progression of symptoms of or the recurrence of and/or treating autoimmune disease or graft-versus-host disease.
